# EUROPEAN PATENT APPLICATION

(11) **EP 4 506 327 A1**
(43) Date of publication of application: **12.02.2025**
(21) Application number: 23784636.5
(22) Date of filing: 23.03.2023
(51) Int. Cl.: C07C 33/36, C07C 43/23, C08K 5/13, C08L 101/00, C08G 63/19, C07C 69/608, C07C 69/635, G02B 5/22, G02B 5/30, C09K 3/00

(54) **ULTRAVIOLET ABSORBENT**

(30) Priority: 08.04.2022 JP 2022064607
(71) Applicant: Osaka Gas Chemicals Co., Ltd., Osaka-shi, Osaka 550-0023 (JP)
(72) Inventor: SUGA, Shogo, Osaka-shi, Osaka 550-0023 (JP); OTA, Yoshiya, Osaka-shi, Osaka 550-0023 (JP); MINOKAMI, Keiko, Osaka-shi, Osaka 550-0023 (JP); MITSUZANE, Mayato, Osaka-shi, Osaka 550-0023 (JP)
(74) Representative: Murgitroyd & Company
(86) International application number: PCT/JP2023/011390
(87) International publication number: WO 2023/195346

(57) **Abstract**

Provided are a novel ultraviolet absorber that has an excellent absorbing property for UV-A or UV-B, furthermore specifically absorbs only ultraviolet ray at a specific wavelength, and is capable of being used for general purposes, and an ultraviolet absorbing resin excellent in bleed-out resistance in addition to the characteristics described above. The ultraviolet absorber is a fluorene compound represented by the general formula (1) or a resin comprising the fluorene compound as a constituent unit.

## Description

### Technical Field

The present invention relates to an ultraviolet absorber excellent in an ultraviolet absorbing property for UV-A and UV-B regions, an ultraviolet absorbing resin, an ultraviolet absorbing coating liquid, an ultraviolet absorbing coated resin sheet, an ultraviolet absorbing coated glass, an ultraviolet absorbing molded article, an ultraviolet absorbing film, a polarizer protective film, a polarizing plate, an image display apparatus, a transport equipment, and a building material component.

### Background Art

Ultraviolet ray is broadly divided, according to its wavelength region, into long-wavelength ultraviolet ray (UV-A; 320 to 400 nm), medium-wavelength ultraviolet ray (UV-B; 280 to 320 nm), and short-wavelength ultraviolet ray (UV-C; 200 to 280 nm). Among them, UV-C is mostly absorbed by the ozone layer before arriving at the Earth's surface. Therefore, a great part of ultraviolet ray reaching the ground is UV-A and UV-B.

Polymer materials differ in ultraviolet wavelength that facilitates inducing deterioration, depending on their types. For example, it is known that many polymer materials such as polyethylene or polyvinyl chloride are markedly deteriorated by UV-B, causing discoloration, reduction in mechanical strength, and the like. Therefore, the addition of an ultraviolet absorber that absorbs UV-B is widely practiced.

Since stability against ultraviolet ray over a longer period has been demanded in recent years, there are growing needs for an ultraviolet absorber that can absorb not only UV-B but UV-A, particularly, in fields such as displays, solar batteries, and automobiles. However, conventional organic ultraviolet absorbers absorb less long-wavelength ultraviolet ray and therefore need to be added in an increased amount. However, the addition of such an ultraviolet absorber in a large amount disadvantageously causes bleed-out or reduction in mechanical strength, heat resistance, or transparency of a resin. Ultraviolet absorbers characterized by absorbing long-wavelength ultraviolet ray absorb even a visible light region of 400 nm or more and are therefore often stained yellow. Thus, ultraviolet absorption performance for a UV-A region and a low staining property are reportedly difficult to achieve.

Among others, optical films that are used for improving viewability in display apparatuses such as a liquid crystal display (LCD) or an organic EL display (OLED) are exposed to ultraviolet ray contained in sunlight as well as ultraviolet ray contained in light sources of the display apparatus for a long time so that deterioration such as yellowing progresses over time, disadvantageously reducing viewability. Along with needs for the miniaturization of display apparatuses, there are demands for excellent ultraviolet absorbers that enable optical films, even if thinned, to exert sufficient ultraviolet absorption performance.

For example, Patent Literature 1 discloses a (meth)acrylic film supplemented with a benzotriazole-based ultraviolet absorber. However, in general, the benzotriazole-based ultraviolet absorber has an insufficient molar absorption coefficient for a UV-A region and thus needs to be added at a high concentration. Hence, a composition containing the benzotriazole-based ultraviolet absorber is disadvantageously susceptible to whitening or embrittlement ascribable to bleed-out.

Patent Literature 2 discloses an ultraviolet absorber consisting of a triazine compound having a strong absorbing property in a wide UV-A region. However, in general, the triazine-based ultraviolet absorber that absorbs ultraviolet ray in a wide wavelength band also absorbs visible light at 400 nm or more and is therefore disadvantageously susceptible to staining.

Patent Literature 3 discloses a method for combining an ultraviolet absorber having maximum absorption at a wavelength of 200 to 300 nm and an ultraviolet absorber having maximum absorption at a wavelength of 320 to 400 nm. This enables both ultraviolet absorption performance at a wavelength of 380 nm and low staining to be achieved. However, a novel ultraviolet absorber that absorbs only ultraviolet ray at a specific wavelength is presumably necessary for further improvement in ultraviolet absorption performance and lower staining.

Patent Literature 4 discloses an ultraviolet absorber composition having a maximum absorption wavelength of 350 nm or more and 400 nm or less, a half width of 55 nm or less, and a molar absorption coefficient of 20000 or more at the maximum absorption wavelength. This can provide a material that absorbs only ultraviolet ray at a specific wavelength and is less stained. Unfortunately, any composition disclosed therein requires a complicated synthesis method and high cost and is therefore difficult to use for general purposes.

It has also been proposed that a polymerizable functional group is added to an ultraviolet absorber so that the ultraviolet absorber itself is cured as a thin-film component in order to prevent the bleed-out of the ultraviolet absorber in an actual environment of usage or its sublimation due to heat at the time of drying or molding.

Patent Literature 5 discloses a resin composition obtained by homopolymerizing a benzotriazole derivative compound, and Patent Literature 6 discloses a compound obtained by adding a reactive functional group to the end of a compound having a triazine skeleton. However, in both the inventions, a functional group that contributes to ultraviolet absorption is conventionally known benzotriazole or triazine. Therefore, problems of these ultraviolet absorbers are insufficient molar absorption coefficient for a UV-A region and easy staining, as shown above.

### Citation List

### Patent Literature

Patent Literature 1: Japanese Patent Laid-Open No. 2019-167411
Patent Literature 2: Japanese Patent Laid-Open No. 2016-102199
Patent Literature 3: Japanese Patent Laid-Open No. 2015-165301
Patent Literature 4: Japanese Patent Laid-Open No. 2010-059235
Patent Literature 5: Japanese Patent Laid-Open No. 2019-034998
Patent Literature 6: Japanese Patent Laid-Open No. 2021-178918

### Summary of Invention

### Technical Problem

An object of the present invention is to provide a novel ultraviolet absorber that has an excellent absorbing property for UV-A or UV-B, furthermore specifically absorbs only ultraviolet ray at a specific wavelength, and is capable of being used for general purposes, and an ultraviolet absorbing resin, an ultraviolet absorbing coating liquid, an ultraviolet absorbing coated resin sheet, an ultraviolet absorbing coated glass, an ultraviolet absorbing molded article, an ultraviolet absorbing film, a polarizer protective film, a polarizing plate, an image display apparatus, a transport equipment, and a building material component excellent in bleed-out resistance in addition to the characteristics described above.

### Solution to Problem

The present inventors have conducted diligent studies to attain the object and found that a fluorene compound has an excellent ultraviolet absorbing property for UV-A or UV-B, furthermore specifically absorbs only ultraviolet ray at a specific wavelength, and is further capable of absorbing ultraviolet ray at various wavelengths by adding an appropriate substituent to the fluorene compound. In addition to this, the present inventors have found that a resin composition having excellent ultraviolet absorption performance can be prepared by adding a polymerizable functional group to the fluorene compound. Based on these findings, the present invention has been completed.

Specifically, the present invention is as follows.
[1] An ultraviolet absorber being
   a fluorene compound represented by the general formula (1) or a resin comprising the fluorene compound as a constituent unit: 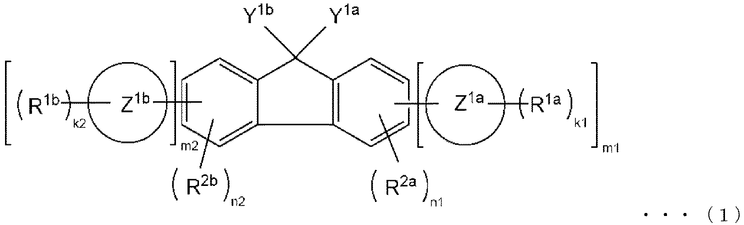 wherein
   Z^{1a} and Z^{1b} each independently represent an arene ring,
   Y^{1a} and Y^{1b} each independently represent a carboxy group, a carboxy ester group, an acid halide group, an acid anhydride group, a hydroxy group, or a group having a polymerizable double bond,
   R^{1a} and R^{1b} each independently represent a halogen atom, an acyl group, a nitro group, a cyano group, a mono- or di-substituted amino group, -R^{A}, -OR^{A}, or -SR^{A} (on the proviso that R^{A} represents a hydrocarbon group),
   k1 and k2 each independently represent an integer of 0 or larger,
   m1 and m2 each independently represent an integer of 0 to 4,
   R^{2a} and R^{2b} each independently represent a halogen atom, a cyano group, or a hydrocarbon group other than an aryl group,
   n1 and n2 each independently represent an integer of 0 to 4, and
   m1 + n1 and m2 + n2 are each independently an integer of 4 or smaller.
[2] The ultraviolet absorber according to [1], wherein
   in the general formula (1), at least one of m1 and m2 is 1 or larger.
[3] The ultraviolet absorber according to [1] or [2], wherein
   in the general formula (1), each of Z^{1a} and Z^{1b} is a condensed polycyclic arene ring, and both m1 and m2 are integers of 1 or larger.
[4] The ultraviolet absorber according to any one of [1] to [3], wherein
   in the general formula (1), Y^{1a} and Y^{1b} are each independently a group represented by the formula (Y1) given below,
   the fluorene compound is dicarboxylic acid or a derivative thereof, and
   the resin is a polyester resin or a polyamide resin comprising the fluorene compound which is dicarboxylic acid as a constituent unit: 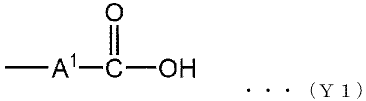 wherein A¹ represents a linear or branched C₁₋₆ alkylene group.
[5] The ultraviolet absorber according to any one of [1] to [3], wherein
   in the general formula (1), Y^{1a} and Y^{1b} are each independently a group represented by the formula (Y2) given below,
   the fluorene compound is diol, and
   the resin is a polyester resin or a polycarbonate resin comprising the fluorene compound which is diol as a constituent unit: 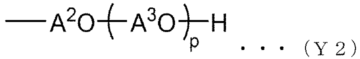 wherein
      A² and A³ each independently represent a linear or branched C₁₋₆ alkylene group, and
      P represents an integer of 0 or larger.
[6] The ultraviolet absorber according to any one of [1] to [3], wherein
   in the general formula (1), Y^{1a} and Y^{1b} are each independently a group represented by the formula (Y3) given below,
   the fluorene compound is diol, and
   the resin is a polyester resin or a polycarbonate resin comprising the fluorene compound which is diol as a constituent unit: 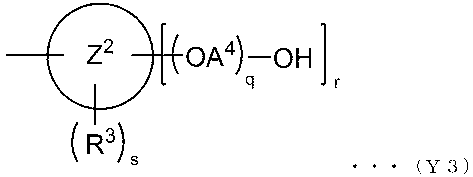 wherein
      Z² represents an arene ring,
      each A⁴ independently represents a linear or branched C₁₋₄ alkylene group,
      each R³ independently represents a substituent,
      q represents an integer of 0 or larger,
      r represents an integer of 1 or larger, and
      s represents an integer of 0 or larger.
[7] The ultraviolet absorber according to [6], wherein
   in the general formula (Y3), Z² is a condensed polycyclic arene ring.
[8] The ultraviolet absorber according to any one of [1] to [3], wherein
   in the general formula (1), Y^{1a} and Y^{1b} are each independently a group represented by the formula (Y4) given below,
   the fluorene compound is di(meth)acrylate, and
   the resin is a (meth)acrylic resin comprising the fluorene compound which is di(meth)acrylate as a constituent unit: 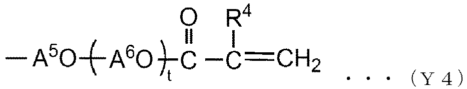 wherein
      A⁵ and A⁶ each independently represent a linear or branched C₁₋₆ alkylene group,
      R⁴ represents a hydrogen atom or a methyl group, and
      t represents an integer of 0 or larger.
[9] The ultraviolet absorber according to any one of [1] to [8], wherein
   a half width which is a wavelength band that indicates 1/2 or more of absorbance at a maximum absorption wavelength is 55 nm or less.
[10] The ultraviolet absorber according to any one of [1] to [9], wherein
   the ultraviolet absorber has a maximum absorption wavelength within a wavelength range of 320 to 400 nm.
[11] The ultraviolet absorber according to any one of [1] to [10], wherein
   a ratio (A₃₈₀/A₄₀₀) of a molar absorption coefficient at 380 nm (A₃₈₀) to a molar absorption coefficient at 400 nm (A₄₀₀) is 50 or more.
[12] The ultraviolet absorber according to any one of [1] to [11], wherein
   a content of the constituent unit derived from the fluorene compound is 1 to 100% by mol based on all constituent units of the resin.
[13] An ultraviolet absorbing composition comprising
   the ultraviolet absorber according to [1], and
   at least one or more compounds selected from a triazine ring-containing compound, a benzotriazole ring-containing compound, and a benzophenone ring-containing compound as a second ultraviolet absorber.
[14] An ultraviolet absorbing resin composition comprising
   the ultraviolet absorber according to any one of [1] to [12], and
   an arbitrary resin, wherein
   a content of the ultraviolet absorber is 0.1 to 10% by mass based on the total amount.
[15] An ultraviolet absorbing coating liquid, comprising
   the ultraviolet absorber according to any one of [1] to [12], the ultraviolet absorbing composition according to [13], or the ultraviolet absorbing resin composition according to [14].
[16] An ultraviolet absorbing coated resin sheet comprising
   a resin sheet and an ultraviolet shielding layer, wherein
   the ultraviolet shielding layer comprises the ultraviolet absorber according to any one of [1] to [12], the ultraviolet absorbing composition according to [13], or the ultraviolet absorbing resin composition according to [14] .
[17] An ultraviolet absorbing coated glass comprising
   glass and an ultraviolet shielding layer, wherein
   the ultraviolet shielding layer comprises the ultraviolet absorber according to any one of [1] to [12], the ultraviolet absorbing composition according to [13], or the ultraviolet absorbing resin composition according to [14] .
[18] An ultraviolet absorbing molded article comprising
   the ultraviolet absorber according to any one of [1] to [12], the ultraviolet absorbing composition according to [13], or the ultraviolet absorbing resin composition according to [14].
[19] An ultraviolet absorbing film comprising
   the ultraviolet absorber according to any one of [1] to [12], the ultraviolet absorbing composition according to [13], or the ultraviolet absorbing resin composition according to [14].
[20] A polarizer protective film comprising
   the ultraviolet absorbing film according to [19].
[21] The polarizer protective film according to [20], wherein
   a spectral transmittance at 350 nm is 5% or less, and
   a b* value in L*a*b* color space (CIELAB) is 0.5 or less.
[22] The polarizer protective film according to [20] or [21], wherein
   a spectral transmittance at 380 nm is 8% or less.
[23] A polarizing plate comprising
   the polarizer protective film according to any one of [20] to [22].
[24] An image display apparatus comprising
   the polarizing plate according to [23].
[25] Transport equipment comprising
   the ultraviolet absorber according to any one of [1] to [12], the ultraviolet absorbing composition according to [13], or the ultraviolet absorbing resin composition according to [14].
[26] A building member comprising
   a member comprising the ultraviolet absorber according to any one of [1] to [12], the ultraviolet absorbing composition according to [13], or the ultraviolet absorbing resin composition according to [14] .

### Advantageous Effects of Invention

The present invention can provide an ultraviolet absorber that has an excellent ultraviolet absorbing property for UV-A or UV-B, specifically absorbs only ultraviolet ray at a specific wavelength, and is capable of being used for general purposes, and an ultraviolet absorbing resin, an ultraviolet absorbing coating liquid, an ultraviolet absorbing coated resin sheet, an ultraviolet absorbing coated glass, an ultraviolet absorbing molded article, an ultraviolet absorbing film, a polarizer protective film, a polarizing plate, an image display apparatus, a transport equipment, and a building material component excellent in bleed-out resistance in addition to the characteristics described above.

### Brief Description of Drawings

[Figure 1A] Figure 1A is a cross-sectional view schematically illustrating a polarizing plate according to one embodiment of the present invention.
[Figure 1B] Figure 1B is a cross-sectional view schematically illustrating a polarizing plate according to another embodiment of the present invention.
[Figure 2A] Figure 2A is a cross-sectional view schematically illustrating an image display apparatus (OLED) according to one embodiment of the present invention.
[Figure 2B] Figure 2B is a cross-sectional view schematically illustrating an image display apparatus (LCD) according to one embodiment of the present invention.
[Figure 3] Figure 3 is a cross-sectional view schematically illustrating a rollable display according to one embodiment of the present invention.
[Figure 4] Figure 4 is a diagram showing the absorbance of ultraviolet absorbing polyester resins obtained in Examples 8 to 13.

### Description of Embodiments

Hereinafter, the mode for carrying out the present invention (hereinafter, referred to as the "present embodiment") will be described in detail. However, the present invention is not limited thereby, and various changes or modifications can be made in the present invention without departing from the spirit of the present invention. In the drawings, the same symbols are used to designate the same or similar components, so that the description is omitted. Positional relationships indicated by the terms "up", "down", "right", "left", and the like are based on the positional relationships shown in the drawings, unless otherwise specified. The dimensional ratios of the drawings are not limited to the ratios shown therein.

In the present embodiment, a term such as "C₂₋₈" means that a group has 2 or more and 8 or less carbon atoms.

### 1. Ultraviolet absorber

The ultraviolet absorber of the present embodiment is a fluorene compound represented by the following general formula (1) or a resin comprising the fluorene compound represented by the following general formula (1) as a constituent unit: 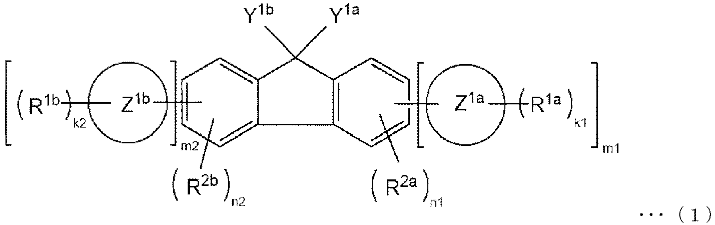 wherein
Z^{1a} and Z^{1b} each independently represent an arene ring,
Y^{1a} and Y^{1b} each independently represent a carboxy group, a hydroxy group, or a group having a polymerizable double bond,
R^{1a} and R^{1b} each independently represent a halogen atom, an acyl group, a nitro group, a cyano group, an amino group, -R^{A}, -OR^{A}, or -SR^{A} (on the proviso that R^{A} represents a hydrocarbon group),
k1 and k2 each independently represent an integer of 0 or larger,
m1 and m2 each independently represent an integer of 0 to 4,
R^{2a} and R^{2b} each independently represent a substituent,
n1 and n2 each independently represent an integer of 0 to 4, and
m1 + n1 and m2 + n2 are each independently an integer of 4 or smaller.

The ultraviolet absorber of the present embodiment, by having the structure of the general formula (1), exhibits an excellent ultraviolet absorbing property for UV-A or UV-B and furthermore specifically absorbs only ultraviolet ray at a specific wavelength. An absorbance wavelength may be adjusted by changing each group shown in the general formula (1) so as to have an arbitrary structure.

The ultraviolet absorber of the present embodiment may be a fluorene compound having a structure represented by the general formula (1) or may be a resin having the structure represented by the general formula (1) as a constituent unit.

Hereinafter, the fluorene compound having a structure represented by the general formula (1) will first be described, and the resin will then be described.

### 1.1. Fluorene compound

The fluorene compound has a structure represented by the general formula (1) and may have arene rings (Z^{1a} and Z^{1b}) and substituents (Y^{1a}, Y^{1b}, R^{1a}, R^{1b}, R^{2a}, and R^{2b}), which will be described below in detail, bonded to a fluorene ring.

### 1.1.1. Group [-Z^{1a}-(R^{1a})ₖ₁] and group [-Z^{1b}-(R^{1b})ₖ₂]

Z^{1a} and Z^{1b} each independently represent an arene ring bonded to a fluorene ring. Examples of such an arene ring include, but are not particularly limited to, monocyclic arene rings such as a benzene ring, condensed polycyclic arene rings (condensed polycyclic aromatic hydrocarbon rings), and ring assembly arene rings (ring assembly polycyclic aromatic hydrocarbon rings). Among them, a condensed polycyclic arene ring is preferred from the viewpoint of a molar absorption coefficient in a UV-A region.

Examples of the condensed polycyclic arene ring include, but are not particularly limited to, a naphthalene ring, an indene ring, an azulene ring, an anthracene ring, a phenanthrene ring, a tetracene ring, a pentacene ring, a benzopyrene ring, a chrysene ring, a dibenzochrysene ring, a pyrene ring, a triphenylene ring, a corannulene ring, a coronene ring, and ovalene ring. Among them, a naphthalene ring is preferred.

Examples of the ring assembly arene ring include, but are not particularly limited to: biarene rings such as a biphenyl ring, a phenylnaphthalene ring, and a binaphthyl ring; and terarene rings such as a terphenyl ring. Among them, a biphenyl ring is preferred.

The substitution positions of Z^{1a} and Z^{1b} on the fluorene ring are not particularly limited and are preferably the 2-position or/and the 7-position in terms of an industrial method for synthesizing the fluorene compound.

When the arene ring represented by Z^{1a} and Z^{1b} each independently is a naphthalene ring, the binding position of the ring Z^{1a} or Z^{1b} to the fluorene skeleton may be the

1-position or the 2-position of the naphthalene ring. Among them, the 1-position of the naphthalene ring is preferred from the viewpoint of a 5% mass loss temperature. The 2-position of the naphthalene ring is particularly preferred from the viewpoint of a molar absorption coefficient.

The number of carbon atoms in each of Z^{1a} and Z^{1b} is preferably 6 to 24, more preferably 6 to 18, further preferably 6 to 12.

The arene rings Z^{1a} and Z^{1b} may each independently have nonreactive or nonpolymerizable substituents R^{1a} and R^{1b}. Examples of the substituents R^{1a} and R^{1b} include, but are not particularly limited to, halogen atoms, acyl groups, a nitro group, a cyano group, mono- or di-substituted amino groups, -R^{A}, -OR^{A}, and -SR^{A}. In this context, R^{A} represents a hydrocarbon group.

Examples of the halogen atom include a fluorine atom, a chlorine atom, a bromine atom, and an iodine atom.

Examples of the acyl group include C₁₋₆ alkylcarbonyl groups such as an acetyl group.

Examples of the mono- or di-substituted amino group include dialkylamino groups and bis(alkylcarbonyl)amino groups. Examples of the dialkylamino group include di-C1-4 alkylamino groups such as a dimethylamino group. Examples of the bis(alkylcarbonyl)amino group include bis(C1-4 alkylcarbonyl)amino groups such as a diacetylamino group.

Examples of the hydrocarbon group represented by R^{A} include alkyl groups, cycloalkyl groups, aryl groups, and aralkyl groups.

Examples of the alkyl group include linear or branched C₁₋₁₀ alkyl groups such as a methyl group, an ethyl group, a propyl group, an isopropyl group, a n-butyl group, an isobutyl group, a s-butyl group, and a t-butyl group. Among them, the alkyl group is preferably a linear or branched C₁₋₆ alkyl group, further preferably a linear or branched C₁₋₄ alkyl group.

Examples of the cycloalkyl group include C₅₋₁₀ cycloalkyl groups such as a cyclopentyl group and a cyclohexyl group.

Examples of the aryl group include C₆₋₁₂ aryl groups such as a phenyl group, alkylphenyl groups, a biphenylyl group, and a naphthyl group. Examples of the alkylphenyl group include mono- to tri-C₁₋₄ alkyl-phenyl groups such as a methylphenyl group (or a tolyl group) and a dimethylphenyl group (or a xylyl group).

Examples of the aralkyl group include C₆₋₁₀ aryl-C₁₋₄ alkyl groups such as a benzyl group and a phenethyl group.

Examples of the group [-OR^{A}] include alkoxy groups, cycloalkyloxy groups, aryloxy groups, and aralkyloxy groups and specifically include groups corresponding to the examples of the hydrocarbon group RA.

Examples of the alkoxy group include linear or branched C₁₋₁₀ alkoxy groups such as a methoxy group, an ethoxy group, a propoxy group, a n-butoxy group, an isobutoxy group, and a t-butoxy group.

Examples of the cycloalkyloxy group include C₅₋₁₀ cycloalkyloxy groups such as a cyclohexyloxy group.

Examples of the aryloxy group include C₆₋₁₀ aryloxy groups such as a phenoxy group.

Examples of the aralkyloxy group include C₆₋₁₀ aryl-C₁₋₄ alkyloxy groups such as a benzyloxy group.

Examples of the group [-SR^{A}] include alkylthio groups, cycloalkylthio groups, arylthio groups, and aralkylthio groups and specifically include groups corresponding to the examples of the hydrocarbon group R^{A}.

Examples of the alkylthio group include C₁₋₁₀ alkylthio groups such as a methylthio group, an ethylthio group, a propylthio group, a n-butylthio group, and a t-butylthio group.

Examples of the cycloalkylthio group include C₅₋₁₀ cycloalkylthio groups such as a cyclohexylthio group.

Examples of the arylthio group include C₆₋₁₀ arylthio groups such as thiophenoxy group.

Examples of the aralkylthio group include C₆₋₁₀ aryl-C₁₋₄ alkylthio groups such as a benzylthio group.

Each of the substituents R^{1a} and R^{1b} is preferably -R^{A}, -OR^{A}, an acyl group, a nitro group, a cyano group, or a substituted amino group, more preferably an alkyl group or an alkoxy group, further preferably a linear or branched C₁₋₆ alkyl group such as a methyl group or a linear or branched C₁₋₄ alkoxy group such as a methoxy group, particularly preferably a linear or branched C₁₋₄ alkyl group such as a methyl group. When each of the groups R^{1a} or R^{1b} is an aryl group, the group R^{1a} and R^{1b} may form ring assembly arene rings together with the rings Z^{1a} or Z^{1b}, respectively.

The numbers k1 and k2 of substitutions represent the numbers of substituents R^{1a} and R^{1b} bonded to the arene rings Z^{1a} and Z^{1b}. The numbers k1 and k2 of substitutions may be selected according to the types of the arene rings Z^{1a} and Z^{1b}. Preferred ranges of the numbers k1 and k2 of substitutions are, in stages, an integer of 0 to 7, an integer of 0 to 6, an integer of 0 to 5, an integer of 0 to 4, an integer of 0 to 3, an integer of 0 to 2, 0 or 1, or 0.

The numbers k1 and k2 of substitutions may be the same as or different from each other. When each of the numbers k1 and k2 of substitutions is 2 or larger, the types of two or more substituents R^{1a} or R^{1b} added on the same ring Z^{1a} or Z^{1b} may be the same as or different from each other. The types of the substituents R^{1a} and R^{1b} added on different rings Z^{1a} or Z^{1b} may be the same as or different from each other. The substitution positions of the groups R^{1a} and R^{1b} are not particularly limited and may be selected according to the types of the rings Z^{1a} and Z^{1b}.

The numbers m1 and m2 of substitutions represent the numbers of groups [-Z^{1a}-(R^{1a})ₖ₁] and [-Z^{1b}-(R^{1b})ₖ₂] (hereinafter, these groups are also referred to as "Z¹-containing groups") bonded to the fluorene ring. The numbers m1 and m2 of substitutions each independently represent an integer of 0 to 4 and are preferably an integer of 1 to 3, more preferably 1 or 2, further preferably 1.

At least one of m1 and m2 is preferably an integer of 1 or larger, more preferably both are integers of 1 or larger, further preferably both are 1, from the viewpoint of a molar absorption coefficient and an absorbance wavelength.

When m1 or m2 is 2 or larger, the types of two or more Z¹-containing substituents added on the same benzene ring of two benzene rings constituting the fluorene skeleton may be the same as or different from each other. The types of two or more Z¹-containing substituents added on different benzene rings of two benzene rings constituting the fluorene skeleton, i.e., the group [-Z^{1a}-(R^{1a} )ₖ₁] and the group [-Z^{1b}-(R^{1b})ₖ₂], may be the same as or different from each other.

### 1.1.2. Group R^{2a} and group R^{2b}

R^{2a} and R^{2b} each independently represent a nonreactive substituent or a nonpolymerizable substituent bonded to a fluorene ring. Such a substituent can be a substituent other than the Z¹-containing groups mentioned above.

Examples of such groups R^{2a} and R^{2b} include hydrocarbon groups such as alkyl groups and cycloalkyl groups (except for aryl groups), halogen atoms such as a fluorine atom, a chlorine atom, a bromine atom, and an iodine atom, and a cyano group.

Examples of the alkyl group include linear or branched C₁₋₁₀ alkyl groups such as a methyl group, an ethyl group, a propyl group, an isopropyl group, a n-butyl group, an isobutyl group, a s-butyl group, and a t-butyl group. Among them, the alkyl group is preferably a linear or branched C₁₋₆ alkyl group, more preferably a linear or branched C₁₋₄ alkyl group such as a methyl group.

The numbers n1 and n2 of substitutions represent the numbers of substituents R^{2a} and R^{2b} bonded to the fluorene ring. The numbers n1 and n2 of substitutions each independently represent an integer of 0 to 4 and are preferably an integer of 0 to 3, more preferably an integer of 0 to 2, further preferably 0 or 1, particularly, 0. n1 and n2 may be the same as or different from each other.

When n1 or n2 is 2 or larger, the types of a plurality of substituents R^{2a} or R^{2b} added on the same benzene ring of two benzene rings constituting the fluorene skeleton may be the same as or different from each other. The types of substituents R^{2a} and R^{2b} added on different benzene rings of two benzene rings constituting the fluorene skeleton may be the same as or different from each other. The substitution positions of R^{2a} and R^{2b} are not particularly limited and can be positions other than the substitution positions of the Z¹-containing groups.

Each of m1 + n1 and m2 + n2 is, for example, an integer of 0 to 4, preferably an integer of 1 to 3, more preferably 1 or 2, further preferably 1. m1 + n1 and m2 + n2 may be the same as or different from each other.

### 1.1.3. Group Y^{1a} and group Y^{1b}

Y^{1a} and Y^{1b} are each independently a 5-membered ring in a fluorene ring, more specifically, a group bonded to carbon at the 9-position. Each of Y^{1a} and Y^{1b} may be a reactive substituent or a polymerizable substituent having a carboxy group, a hydroxy group, or a polymerizable double bond. Examples of such Y^{1a} and Y^{1b} include, but are not particularly limited to, groups of the formula (Y1) given below, the formula (Y2) given below, the formula (Y3) given below, and the formula (Y4) given below.

### 1.1.3.1. Formula (Y1): dicarboxylic acid and derivative thereof

The formula (Y1) is shown below. When each of Y^{1a} and Y^{1b} is represented by the formula (Y1), the fluorene compound of the present embodiment may be dicarboxylic acid or a derivative thereof. 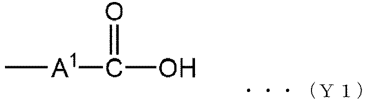 wherein A¹ represents a linear or branched alkylene group.

In this context, examples of the derivative of dicarboxylic acid include ester forming derivatives such as alkyl ester of the formula (Y1) (e.g., lower alkyl ester such as methyl ester and ethyl ester), acid halide (e.g., acid chloride), and acid anhydride.

Examples of the linear or branched alkylene group represented by A¹ include linear or branched C₁₋₆ alkylene groups such as a methylene group, an ethylene group, a propylene group, a trimethylene group, a 1,2-butanediyl group, a 1,3-butanediyl group, and a tetramethylene group.

The number of carbon atoms in A¹ is preferably 1 to 10, more preferably 1 to 6, further preferably 1 to 4.

Use of such A¹ attains an excellent ultraviolet absorbing property for UV-A or UV-B, enables only ultraviolet ray at a specific wavelength to be specifically absorbed, and tends to more improve compatibility with another resin to be mixed.

Typical examples of the compound as the dicarboxylic acid or the derivative thereof in which each of Y^{1a} and Y^{1b} is represented by the formula (Y1) include, but are not particularly limited to, 9,9-bis(2-carboxyethyl)fluorene, 9,9-bis(2-carboxypropyl)fluorene, 9,9-bis(carboxy C4-6 alkyl)fluorene, 9,9-bis(2-carboxyethyl)2,7-diphenylfluorene, 9,9-bis(2-carboxypropyl)2,7-diphenylfluorene, 9,9-bis(carboxy C4-6 alkyl)2,7-diphenylfluorene, 9,9-bis(2-carboxyethyl)2,7-di(2-naphthyl)fluorene, 9,9-bis(2-carboxypropyl)2,7-di(2-naphthyl)fluorene, 9,9-bis(carboxy C4-6 alkyl)2,7-di(2-naphthyl)fluorene, 9,9-bis(2-carboxyethyl)2,7-di(1-naphthyl)fluorene, 9,9-bis(2-carboxypropyl)2,7-di(1-naphthyl)fluorene, and 9,9-bis(carboxy C4-6 alkyl)2,7-di(1-naphthyl)fluorene. These fluorene dicarboxylic acid components may be used singly or in combination of two or more thereof.

### 1.1.3.2. Formula (Y2) : diol

The formula (Y2) is shown below. When each of Y^{1a} and Y^{1b} is represented by the formula (Y2), the fluorene compound of the present embodiment may be diol. 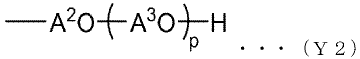 wherein A² and A³ each independently represent a linear or branched alkylene group, and p represents an integer of 0 or larger.

Examples of the linear or branched alkylene group represented by each of A² and A³ include linear or branched C₁₋₆ alkylene groups such as a methylene group, an ethylene group, a propylene group, a trimethylene group, a 1,2-butanediyl group, a 1,3-butanediyl group, and a tetramethylene group. Among them, the linear or branched alkylene group is preferably an ethylene group or a propylene group, particularly preferably an ethylene group.

The number of carbon atoms in each of A² and A³ is preferably 1 to 6, more preferably 2 to 6, further preferably 2 to 3.

Use of such A² and A³ attains an excellent ultraviolet absorbing property for UV-A or UV-B, enables only ultraviolet ray at a specific wavelength to be specifically absorbed, and tends to more improve compatibility with another resin to be mixed.

The number p of repeats of the oxyalkylene group - (A³O)- is an integer of 0 or larger and can be selected from a range on the order of, for example, 0 to 20. A preferred range thereof is, in stages, 0 to 15, 0 to 10, 0 to 8, 0 to 5, 0 to 3, 0 to 2, or 0 to 1, particularly, 0. The number p of repeats may be an average value (or an arithmetic average value), i.e., an average number of moles of addition, and the range thereof is the same as that of the integers described above, including preferred forms. When p is 2 or larger, the types of two or more A³ moieties in the (poly)oxyalkylene group -(A³O)ₚ- may be the same as or different from each other and are preferably the same as each other.

The total number of the numbers p of repeats in Y^{1a} and Y^{1b}, i.e., the total number of oxyalkylene groups (or an average value of the total numbers of moles of addition) in one molecule of a diol compound represented by the formula (1) [hereinafter, also simply referred to as the total number of p] can be selected from a range on the order of, for example, 0 to 30. A preferred range thereof is, in stages, 0 to 20, 0 to 10, 0 to 6, 0 to 4, or 0 to 2, further preferably 0 to 1, particularly 0. The total number of p may be an integer or may be an average value of the total numbers of moles.

Typical examples of the compound as the diol in which each of Y^{1a} and Y^{1b} is represented by the formula (Y2) include, but are not particularly limited to, diol compounds in which each of m1 and m2 is 1, each of Z^{1a} and Z^{1b} is a C₆₋₁₂ arene ring such as a benzene ring, a naphthalene ring, or a biphenyl ring, A² is a linear or branched C₁₋₆ alkylene group, A³ is a linear or branched C₂₋₄ alkylene group, and p is 0 or 1 to 10; preferably diol compounds in which each of m1 and m2 is 1, each of Z^{1a} and Z^{1b} is a benzene ring or a naphthalene ring, A² is a linear or branched C₁₋₄ alkylene group, A³ is a linear or branched C₂₋₃ alkylene group such as an ethylene group or a propylene group, and p is 0 or 1 to 6.

Among them, a diol compound is further preferred in which each of m1 and m2 is 1, each of Z^{1a} and Z^{1b} is a naphthalene ring, A² is a linear or branched C₂₋₄ alkylene group, A³ is an ethylene group, and p is 0 or 1.

Among them, 9,9-bis(3-hydroxypropyl)-dinaphthylfluorene such as 9,9-bis(3-hydroxypropyl)-di(1-naphthyl)fluorene or 9,9-bis(3-hydroxypropyl)-di(2-naphthyl)fluorene is preferred. Particularly, 9,9-bis(3-hydroxypropyl)-2,7-dinaphthylfluorene such as 9,9-bis(3-hydroxypropyl)-2,7-di(2-naphthyl)fluorene is preferred.

### 1.1.3.3. Formula (Y3): diol

Y^{1a} and Y^{1b} are shown below. When each of Y^{1a} and Y^{1b} is represented by the formula (Y3), the fluorene compound of the present embodiment may be diol. 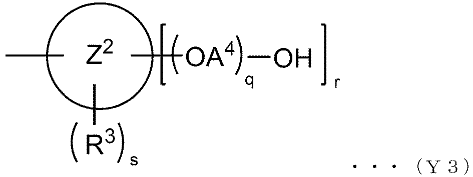 wherein Z² represents an arene ring, each A⁴ independently represents a linear or branched alkylene group, each R³ independently represents a substituent, q represents an integer of 0 or larger, r represents an integer of 1 or larger, and s represents an integer of 0 or larger.

In the formula (Y3), examples of the arene ring represented by Z² include monocyclic arene rings such as a benzene ring, condensed polycyclic arene rings (condensed polycyclic aromatic hydrocarbon rings), and ring assembly arene rings (ring assembly polycyclic aromatic hydrocarbon rings). Among them, a condensed polycyclic arene ring is preferred from the viewpoint of a molar absorption coefficient in a UV-A region.

Examples of the condensed polycyclic arene ring include, but are not particularly limited to, a naphthalene ring, an indene ring, an azulene ring, an anthracene ring, a phenanthrene ring, a tetracene ring, a pentacene ring, a benzopyrene ring, a chrysene ring, a dibenzochrysene ring, a pyrene ring, a triphenylene ring, a corannulene ring, a coronene ring, and an ovalene ring. Among them, a naphthalene ring is preferred.

Examples of the ring assembly arene ring include, but are not particularly limited to: biarene rings such as a biphenyl ring, a phenylnaphthalene ring, and a binaphthyl ring; and terarene rings such as a terphenyl ring. Among them, a biphenyl ring is preferred.

The arene ring Z² may have a nonreactive or nonpolymerizable substituent R³. Examples of the substituent R³ include hydrocarbon groups such as alkyl groups, halogen atoms such as a fluorine atom, a chlorine atom, and a bromine atom, and a cyano group.

Examples of the alkyl group include linear or branched C₁₋₁₀ alkyl groups such as a methyl group, an ethyl group, a propyl group, an isopropyl group, a n-butyl group, an isobutyl group, a s-butyl group, and a t-butyl group. Among them, the alkyl group is preferably a linear or branched C₁₋₆ alkyl group, more preferably a linear or branched C₁₋₄ alkyl group such as a methyl group.

The number s of substitutions represents the number of substituents R³ bonded to the arene ring Z². The number s of substitutions is, for example, an integer of 0 to 4, preferably an integer of 0 to 2, further preferably 0 or 1, particularly 0. The substitution position of R³ is not particularly limited and can be a position other than the substitution position of the group [-(OA⁴)_{q}-OH].

Examples of the linear or branched alkylene group represented by A⁴ include linear or branched C₁₋₆ alkylene groups such as a methylene group, an ethylene group, a propylene group, a trimethylene group, a 1,2-butanediyl group, a 1,3-butanediyl group, and a tetramethylene group. Among them, the alkylene group A⁴ is preferably a linear or branched C₂₋₆ alkylene group, further preferably a linear or branched C₂₋₃ alkylene group, among which an ethylene group or a propylene group is preferred, and an ethylene group is particularly preferred.

A preferred range of the number q of repeats of the oxyalkylene group -(OA⁴)- is, in stages, 0 to 20, 0 to 15, 0 to 10, 0 to 8, 0 to 5, 0 to 3, 0 to 2, 0 to 1, or 0. The number q of repeats may be an average value(or an arithmetic average value), i.e., an average number of moles of addition, and the range thereof is the same as that of the integers described above, including preferred forms. When q is 2 or larger, the types of two or more A⁴ moieties in the (poly)oxyalkylene group-(OA⁴)_{q}- may be the same as or different from each other and are preferably the same as each other.

The total number of the numbers q of repeats in Y^{1a} and Y^{1b}, i.e., the total number of oxyalkylene groups (or an average value of the total numbers of moles of addition) in one molecule of a diol compound represented by the formula (1) [hereinafter, also simply referred to as the total number of q] can be selected from a range on the order of, for example, 0 to 30. A preferred range thereof is, in stages, 0 to 20, 0 to 10, 0 to 6, 0 to 4, or 0 to 2, further preferably 0 to 1, particularly 0.

The total number of q may be an integer or may be an average value of the total numbers of moles.

The number r of substitutions represents the number of groups [-(OA⁴)_{q}-OH] bonded to the arene ring Z². The number r of substitutions is, for example, an integer of 1 to 4, preferably an integer of 1 to 2, further preferably 1. The substitution position of -(OA⁴)_{q}-OH is not particularly limited and can be a position other than the substitution position of R³.

Typical examples of the compound as the diol in which each of Y^{1a} and Y^{1b} is represented by the formula (Y3) include, but are not particularly limited to, diol compounds in which each of m1 and m2 is 0 or 1, each of Z^{1a}, Z^{1b} and Z² is a C₆₋₁₂ arene ring such as a benzene ring, a naphthalene ring, or a biphenyl ring, A⁴ is a linear or branched C₂₋₄ alkylene group, and q is 0 or 1 to 10; preferably diol compounds in which each of m1 and m2 is 0 or 1, each of Z^{1a}, Z^{1b} and Z² is a benzene ring or a naphthalene ring, A⁴ is a linear or branched C₂₋₃ alkylene group such as an ethylene group or a propylene group, and q is 0 or 1 to 6.

Among them, 9,9-bis[hydroxy(poly)alkoxyphenyl]fluorene typified by 9,9-bis[4-(2-hydroxyethoxy)phenyl]fluorene, 9,9-bis[4-(2-(2-hydroxyethoxy)ethoxy)phenyl]fluorene, or 9,9-bis[4-(2-hydroxypropoxy)phenyl]fluorene, or 9,9-bis[hydroxy(poly)alkoxynaphthyl]fluorene typified by 9,9-bis[6-(2-hydroxyethoxy)-2-naphthyl]fluorene, 9,9-bis[5-(2-hydroxyethoxy)-1-naphthyl]fluorene, 9,9-bis[6-(2-(2-hydroxyethoxy)ethoxy)-2-naphthyl]fluorene, or 9,9-bis[6-(2-hydroxypropoxy)-2-naphthyl]fluorene is preferred.

### 1.1.3.4. Formula (Y4): di(meth)acrylate

The formula (Y4) is shown below. When each of Y^{1a} and Y^{1b} is represented by the formula (Y4), the fluorene compound of the present embodiment may be di(meth)acrylate. 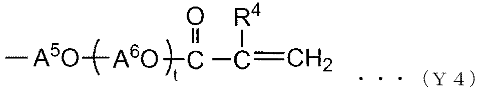 wherein A⁵ and A⁶ each independently represent a linear or branched alkylene group, R⁴ represents a hydrogen atom or a methyl group, and t represents an integer of 0 or larger.

Examples of the linear or branched alkylene group represented by each of A⁵ and A⁶ include linear or branched C₁₋₆ alkylene groups such as a methylene group, an ethylene group, a propylene group, a trimethylene group, a 1,2-butanediyl group, a 1,3-butanediyl group, and a tetramethylene group. Among them, each of the alkylene groups A⁵ and A⁶ is preferably a linear or branched C₂₋₆ alkylene group, further preferably a linear or branched C₂₋₃ alkylene group, among which an ethylene group or a propylene group is preferred, and an ethylene group is preferred.

The number t of repeats of the oxyalkylene group - (A⁶O)- can be selected from a range on the order of, for example, 0 to 20. A preferred range thereof is, in stages, 0 to 15, 0 to 10, 0 to 8, 0 to 5, 0 to 3, 0 to 2, or 0 to 1, particularly, 0. The number t of repeats may be an average value (or an arithmetic average value), i.e., an average number of moles of addition, and the range thereof is the same as that of the integers described above, including preferred forms. When t is 2 or larger, the types of two or more A⁶ moieties in the (poly)oxyalkylene group -(A⁶O)ₜ- may be the same as or different from each other and are preferably the same as each other.

The substituent represented by R⁴ is a hydrogen atom or a methyl group. In the case of radical-polymerizing the ultraviolet absorber of the present embodiment for use, a hydrogen atom is preferred because of its excellent reactivity with radicals.

Typical examples of the compound as the di(meth)acrylate in which each of Y^{1a} and Y^{1b} is represented by the formula (Y4) include, but are not particularly limited to, 9,9-bis((meth)acryloyl)fluorene, 9,9-bis((meth)acryloyloxyethyl)fluorene, 9,9-bis((meth)acryloyloxymethyl)fluorene, 9,9-bis((meth)acryloyloxypropyl)2,7-diphenylfluorene, and 9,9-bis((meth)acryloyloxypropyl)2,7-di(2-naphthyl)fluorene. These di(meth)acrylate components may each be used singly, or two or more thereof may be combined.

### 1.2. Resin

Subsequently, the resin comprising the fluorene compound represented by the general formula (1) as a constituent unit (hereinafter, also simply referred to as a "constituent unit F") will be described in detail. The resin of the present embodiment, by comprising the fluorene compound represented by the general formula (1) as a constituent unit, has an excellent absorbing property for UV-A or UV-B and furthermore is capable of specifically absorbing only ultraviolet ray at a specific wavelength.

Examples of such a resin of the present embodiment include, but are not particularly limited to, polyester resins, polycarbonate resins, polyamide resins, and acrylic resins. Hereinafter, each resin will be described in detail. However, the resin of the present embodiment is not limited to those described below as long as the resin comprises the constituent unit F.

### 1.2.1. Polyester resin

The polyester resin is a resin of diol and dicarboxylic acid sequentially polymerized to form ester bonds, and has the constituent unit F derived from the fluorene compound represented by the general formula (1) as at least one or both of the diol and the dicarboxylic acid. Such a polyester resin comprising the constituent unit F is also referred to as an "ultraviolet absorbing polyester resin" for discrimination from other polyester resins.

The content of the constituent unit F contained in the ultraviolet absorbing polyester resin is preferably 1 to 100% by mol, more preferably 5 to 80% by mol, further preferably 10 to 60% by mol, in terms of a molar ratio based on all constituent units. When the content ratio of the constituent unit F falls within the range described above, an ultraviolet absorbing property and ultraviolet absorption specificity at a specific wavelength tend to be more improved.

### 1.2.1.1. Diol component

All the diol components of the ultraviolet absorbing polyester resin may be the constituent unit F, or the constituent unit F serving as a diol component may be combined with an additional diol component. Examples of the constituent unit F serving as a diol component include, but are not particularly limited to, a constituent unit derived from diol in which each of Y^{1a} and Y^{1b} is represented by the formula (Y2), and a constituent unit derived from diol in which each of Y^{1a} and Y^{1b} is represented by the formula (Y3). These diols may each be used singly, or two or more thereof may be used in combination.

Examples of the additional diol component include, but are not particularly limited to, at least one diol component selected from the group consisting of aliphatic diol, alicyclic diol, and aromatic diol.

Examples of the aliphatic diol can include C₂₋₈ alkanediol such as ethylene glycol, 1,2-propanediol, 1,3-propanediol, 1,2-butanediol, 1,3-butanediol, 1,4-butanediol, 1,3-pentanediol, 1,4-pentanediol, 1,5-pentanediol, neopentyl glycol, and 1,6-hexanediol, and polyalkanediol (e.g., di- or tri-C₂₋₄ alkanediol such as diethylene glycol, dipropylene glycol, and triethylene glycol). Among them, ethylene glycol is particularly preferred because mechanical characteristics such as elongation at break and flexibility can be improved.

Examples of the alicyclic diol include: cycloalkanediol such as cyclohexanediol; bis(hydroxyalkyl)cycloalkane such as cyclohexanedimethanol; and hydrogenated products of aromatic diol listed later, such as hydrogenated bisphenol A.

Examples of the aromatic diol include: dihydroxyarene such as hydroquinone and resorcinol; araliphatic diol such as benzenedimethanol; bisphenols such as bisphenol A, bisphenol F, bisphenol AD, bisphenol C, bisphenol G, and bisphenol S; and biphenols such as p,p'-biphenol.

These additional diol components may each be used singly, or two or more thereof may be combined.

### 1.2.1.2. Dicarboxylic acid

All the dicarboxylic acid components of the ultraviolet absorbing polyester resin may be the constituent unit F, or the constituent unit F serving as a dicarboxylic acid component may be combined with an additional dicarboxylic acid component. Examples of the constituent unit F serving as a dicarboxylic acid component include, but are not particularly limited to, a constituent unit derived from dicarboxylic acid in which each of Y^{1a} and Y^{1b} is represented by the formula (Y1) . These dicarboxylic acids may each be used singly, or two or more thereof may be used in combination.

Examples of the additional dicarboxylic acid component include, but are not particularly limited to, at least one dicarboxylic acid component selected from the group consisting of aliphatic dicarboxylic acid, alicyclic dicarboxylic acid, and aromatic dicarboxylic acid.

Examples of the aliphatic dicarboxylic acid include, but are not particularly limited to, alkanedicarboxylic acid (e.g., C₄₋₁₄ alkanedicarboxylic acid such as succinic acid, adipic acid, sebacic acid, and decanedicarboxylic acid, preferably C₆₋₁₂ alkanedicarboxylic acid), and unsaturated aliphatic dicarboxylic acid (e.g., C₂₋₁₀ alkene-dicarboxylic acid such as maleic acid, fumaric acid, and itaconic acid). The aliphatic dicarboxylic acid is preferably alkanedicarboxylic acid.

Examples of the alicyclic dicarboxylic acid component include, but are not particularly limited to, cycloalkanedicarboxylic acid (e.g., C₅₋₁₀ cycloalkanedicarboxylic acid such as 1,3-cyclohexanedicarboxylic acid and 1,4-cyclohexanedicarboxylic acid), di- or tri-cycloalkanedicarboxylic acid (e.g., decalindicarboxylic acid, norbornanedicarboxylic acid, adamantanedicarboxylic acid, and tricyclodecanedicarboxylic acid), cycloalkenedicarboxylic acid (e.g., C₅₋₁₀ cycloalkenedicarboxylic acid such as cyclohexenedicarboxylic acid), and di- or tri-cycloalkenedicarboxylic acid (e.g., norbornenedicarboxylic acid).

Examples of the aromatic dicarboxylic acid component include, but are not particularly limited to, monocyclic aromatic dicarboxylic acid [e.g., C₆₋₁₀ arenedicarboxylic acid such as phthalic acid, terephthalic acid, isophthalic acid, and alkylisophthalic acid (e.g., C₁₋₄ alkylisophthalic acid such as 4-methylisophthalic acid)], condensed polycyclic aromatic dicarboxylic acid [e.g., condensed polycyclic C₁₀₋₂₄ arene-dicarboxylic acid such as naphthalenedicarboxylic acid (e.g., 1,5-naphthalenedicarboxylic acid, 1,6-naphthalenedicarboxylic acid, 1,7-naphthalenedicarboxylic acid, 1,8-naphthalenedicarboxylic acid, and 2,6-naphthalenedicarboxylic acid), anthracenedicarboxylic acid, and phenanthrenedicarboxylic acid, preferably condensed polycyclic C₁₀₋₁₆ arene-dicarboxylic acid, further preferably condensed polycyclic C₁₀₋₁₄ arene-dicarboxylic acid], arylarenedicarboxylic acid [e.g., C₆₋₁₀ aryl-C₆₋₁₀ arenedicarboxylic acid such as biphenyldicarboxylic acid (e.g., 2,2'-biphenyldicarboxylic acid, and 4,4'-biphenyldicarboxylic acid)], diarylalkanedicarboxylic acid [e.g., di-C₆₋₁₀ aryl-C₁₋₆ alkane-dicarboxylic acid such as diphenylalkanedicarboxylic acid (e.g., 4,4'-diphenylmethanedicarboxylic acid)], and diaryl ketone dicarboxylic acid [e.g., di-C₆₋₁₀ aryl ketone-dicarboxylic acid such as diphenyl ketone dicarboxylic acid (e.g., 4.4'-diphenyl ketone dicarboxylic acid)].

The dicarboxylic acid component contained in the ultraviolet absorbing polyester resin is not only free carboxylic acid but includes ester-forming derivatives of dicarboxylic acid, for example, ester [e.g., alkyl ester [e.g., lower alkyl ester (e.g., C₁₋₄ alkyl ester, particularly, C₁₋₂ alkyl ester) such as methyl ester or ethyl ester]], acid halide (e.g., acid chloride), and acid anhydride. These dicarboxylic acid components may each be used singly, or two or more thereof may be used in combination.

### 1.2.1.3. Method for preparing polyester resin

The ultraviolet absorbing polyester resin can be prepared through the reaction of the dicarboxylic acid component with the diol component. The method for producing the polyester resin is not particularly limited, and the polyester resin may be prepared by a common method, for example, a transesterification method, a melt polymerization method such as a direct polymerization method, a solution polymerization method, or an interfacial polymerization method. In the polymerization reaction, a transesterification catalyst, a polycondensation catalyst, a heat stabilizer, a light stabilizer, a polymerization modifier, or the like may be used.

Examples of the transesterification catalyst include, but are not particularly limited to, compounds (alkoxide, organic acid salt, inorganic acid salt, metal oxide, etc.) of alkaline earth metals (magnesium, calcium, barium, etc.) or transition metals (manganese, zinc, cobalt, titanium, etc.). Among them, manganese acetate, calcium acetate, or the like can be suitably used.

Examples of the type of the polycondensation catalyst can include, but are not particularly limited to, compounds of alkaline earth metals, transition metals, group 13 metals of the periodic table (aluminum, etc.), group 14 metals of the periodic table (germanium, etc.), or group 15 metals of the periodic table (antimony, etc.), more specifically, germanium compounds such as germanium dioxide, germanium hydroxide, germanium oxalate, germanium tetraethoxide, and germanium n-butoxide, antimony compounds such as antimony trioxide, antimony acetate, and antimony ethylene glycolate, and titanium compounds such as tetra-n-propyl titanate, tetraisopropyl titanate, tetra-n-butyl titanate, titanium oxalate, and titanium potassium oxalate. These catalysts may each be used singly, or two or more types thereof may be used as a mixture.

Examples of the heat stabilizer can include, but are not particularly limited to, phosphorus compounds such as trimethyl phosphate, triethyl phosphate, triphenyl phosphate, phosphorus acid, trimethyl phosphite, and triethyl phosphite.

In the reaction, the ratios of the dicarboxylic acid component and the diol component used can be selected from the same ranges as above. If necessary, a predetermined component may be used in excess. For example, a diol component, such as ethylene glycol, capable of being distilled off from the reaction system may be used in excess over the ratio of the unit to be introduced in the polyester resin. The reaction may be performed in the presence or absence of a solvent.

The reaction can be performed in an inert gas (nitrogen, helium, etc.) atmosphere. The reaction can also be performed under reduced pressure (e.g., on the order of 1 × 10² to 1 × 10⁴ Pa). The reaction temperature depends on a polymerization method, and the reaction temperature in a melt polymerization method, for example, may be on the order of 150 to 300°C, preferably 180 to 290°C, further preferably 200 to 280°C.

### 1.2.1.3. Physical properties of polyester resin

The glass transition temperature of the ultraviolet absorbing polyester resin is preferably 90 to 190°C, more preferably 100 to 180°C, further preferably 110 to 170°C. When the glass transition temperature is 90°C or higher, the heat resistance of the ultraviolet absorbing polyester resin tends to be more improved. When the glass transition temperature is 190°C or lower, the drawability of the ultraviolet absorbing polyester resin tends to be more improved. The glass transition temperature can be measured by a method described in Examples mentioned later.

The weight-average molecular weight of the ultraviolet absorbing polyester resin is preferably 30000 to 200000, more preferably 35000 to 150000, further preferably 40000 to 110000. When the weight-average molecular weight falls within the range described above, the ultraviolet absorbing polyester resin has a long molecular chain, mechanical characteristics such as elongation at break and flexibility tend to be more improved, and drawability tends to be more improved. In the present embodiment, the weight-average molecular weight can be measured by gel permeation chromatography (GPC) based on polystyrene. More specifically, the weight-average molecular weight can be measured by, for example, a method described in Examples mentioned later.

### 1.2.2. Polycarbonate resin

The polycarbonate resin is a resin of diol and phosgene sequentially polymerized to form carbonate groups, and at least some diols have the constituent unit F derived from the fluorene compound represented by the general formula (1). Such a polycarbonate resin comprising the constituent unit F is also referred to as an "ultraviolet absorbing polycarbonate resin" for discrimination from other polycarbonates.

The molar ratio of the constituent unit F contained in the ultraviolet absorbing polycarbonate resin is preferably 1 to 100% by mol, more preferably 5 to 80% by mol, further preferably 10 to 60% by mol, based on all diol units. When the content ratio of the constituent unit F falls within the range described above, an ultraviolet absorbing property and ultraviolet absorption specificity at a specific wavelength tend to be more improved.

### 1.2.2.1. Diol

All the diol components of the ultraviolet absorbing polycarbonate resin may be the constituent unit F, or the constituent unit F serving as a diol component may be combined with an additional diol component. Examples of the constituent unit F serving as a diol component include, but are not particularly limited to, a constituent unit derived from diol in which each of Y^{1a} and Y^{1b} is represented by the formula (Y2), and a constituent unit derived from diol in which each of Y^{1a} and Y^{1b} is represented by the formula (Y3). These diols may each be used singly, or two or more thereof may be used in combination.

Examples of the additional diol component include, but are not particularly limited to, at least one diol component selected from the group consisting of aliphatic diol, alicyclic diol, and aromatic diol.

Examples of the aliphatic diol can include C₂₋₈ alkanediol such as ethylene glycol, 1,2-propanediol, 1,3-propanediol, 1,2-butanediol, 1,3-butanediol, 1,4-butanediol, 1,3-pentanediol, 1,4-pentanediol, 1,5-pentanediol, neopentyl glycol, and 1,6-hexanediol, and polyalkanediol (e.g., di- or tri-C₂₋₄ alkanediol such as diethylene glycol, dipropylene glycol, and triethylene glycol). Among them, ethylene glycol is particularly preferred because mechanical characteristics such as elongation at break and flexibility can be improved.

Examples of the alicyclic diol include: cycloalkanediol such as cyclohexanediol; bis(hydroxyalkyl)cycloalkane such as cyclohexanedimethanol; and hydrogenated products of aromatic diol listed later, such as hydrogenated bisphenol A.

Examples of the aromatic diol include: dihydroxyarene such as hydroquinone and resorcinol; araliphatic diol such as benzenedimethanol; bisphenols such as bisphenol A, bisphenol F, bisphenol AD, bisphenol C, bisphenol G, and bisphenol S; and biphenols such as p,p'-biphenol.

These additional diol components may each be used singly, or two or more thereof may be combined.

### 1.2.2.2. Method for preparing polycarbonate resin

The ultraviolet absorbing polycarbonate resin can be produced by reacting (polymerizing or condensing) a diol component with phosgene or carbonic acid diester (diphenyl carbonate, etc.) by a common method, for example, a phosgene method (solvent method) or a transesterification method (melting method). The diol component can comprise at least the ultraviolet absorber represented by the general formula (1) and may optionally comprise other diol components. Among these methods, a transesterification method is preferred, for example, because no solvent is necessary.

In the transesterification method, the ratio of the carbonic acid diester may be on the order of, for example, 0.8 to 1.5 mol, preferably 0.9 to 1.2 mol, based on 1 mol of the diol component.

The transesterification reaction may be performed in the presence of a catalyst. Examples of the catalyst include various catalysts for use in transesterification reaction, for example, nitrogen-containing compounds and metal compounds.

Examples of the nitrogen-containing compound include quaternary ammonium hydroxide (e.g., tetraalkylammonium hydroxide such as tetramethylammonium hydroxide, tetraethylammonium hydroxide, and tetrabutylammonium hydroxide; and trialkyl-aralkylammonium hydroxide such as trimethylbenzylammonium hydroxide); and tertiary amine (e.g., trialkylamine such as trimethylamine and triethylamine; dimethyl-aralkylamine such as dimethylbenzylamine; and triarylamine such as triphenylamine).

Examples of the metal compound that can be used include metal compounds containing metals such as alkali metals (sodium, etc.), alkaline earth metals (magnesium, calcium, barium, etc.), transition metals (manganese, zinc, cadmium, lead, cobalt, titanium, etc.), group 13 metals of the periodic table (aluminum, etc.), group 14 metals of the periodic table (germanium, etc.), or group 15 metals of the periodic table (antimony, etc.), and more specifically include alkoxide, organic acid salts (acetate, propionate, etc.), inorganic acid salts (borate, carbonate, etc.), oxide, and hydroxide of these metals.

These catalysts can each be used singly, or two or more thereof can be used in combination. Among these catalysts, a nitrogen-containing compound such as quaternary ammonium hydroxide is preferred. Particularly, tetraalkylammonium hydroxide such as tetramethylammonium hydroxide is preferred. The amount of the catalyst used may be on the order of, for example, 0.01 × 10⁻⁴ to 100 × 10⁻⁴ mol, preferably 0.1 × 10⁻⁴ to 40 × 10⁻⁴ mol, based on 1 mol of the diol component.

The reaction may be performed, if necessary, in the presence of an additive such as a stabilizer (antioxidant, heat stabilizer, etc.).

The reaction can usually be performed in an inert gas (nitrogen; noble gas such as helium or argon; etc.) atmosphere. The reaction can also be performed under reduced pressure (e.g., on the order of 1 × 10² to 1 × 10⁴ Pa). The reaction temperature can be selected depending on a polymerization method, and the reaction temperature in a transesterification method, for example, may be on the order of 150 to 320°C, preferably 200 to 310°C, further preferably 250 to 300°C. Particularly, in the case of using diphenyl carbonate as carbonic acid diester, polycondensation is effectively performed while phenol is distilled off under reduced pressure at a high temperature.

### 1.2.2.3. Physical properties of polycarbonate resin

The glass transition temperature of the ultraviolet absorbing polycarbonate resin is preferably 90 to 190°C, more preferably 100 to 180°C, further preferably 110 to 170°C. When the glass transition temperature is 90°C or higher, the heat resistance of the ultraviolet absorbing polycarbonate resin tends to be more improved. When the glass transition temperature is 190°C or lower, the drawability of the ultraviolet absorbing polycarbonate resin tends to be more improved. The glass transition temperature can be measured by a method described in Examples mentioned later.

The weight-average molecular weight of the ultraviolet absorbing polycarbonate resin is preferably 30000 to 200000, more preferably 35000 to 150000, further preferably 40000 to 110000. When the weight-average molecular weight falls within the range described above, the ultraviolet absorbing polycarbonate resin has a long molecular chain, mechanical characteristics such as elongation at break and flexibility tend to be more improved, and drawability tends to be more improved. In the present embodiment, the weight-average molecular weight can be measured by gel permeation chromatography (GPC) based on polystyrene. More specifically, the weight-average molecular weight can be measured by, for example, a method described in Examples mentioned later.

### 1.2.3. Polyamide resin

The polyamide resin is a resin of diamine and dicarboxylic acid sequentially polymerized to form amide bonds, and at least some dicarboxylic acids have the constituent unit F derived from the fluorene compound represented by the general formula (1). Such a polyamide resin comprising the constituent unit F is also referred to as an "ultraviolet absorbing polyamide resin" for discrimination from other polyamides.

The molar ratio of the constituent unit F contained in the ultraviolet absorbing polyamide resin is preferably 1 to 100% by mol, more preferably 5 to 80% by mol, further preferably 10 to 60% by mol, based on all dicarboxylic acid units. When the content ratio of the ultraviolet absorber falls within the range described above, an ultraviolet absorbing property and ultraviolet absorption specificity at a specific wavelength tend to be more improved.

### 1.2.3.1. Dicarboxylic acid

All the dicarboxylic acid components of the ultraviolet absorbing polyamide resin may be the constituent unit F, or the constituent unit F serving as a dicarboxylic acid component may be combined with an additional dicarboxylic acid component. Examples of the constituent unit F serving as a dicarboxylic acid component include, but are not particularly limited to, a constituent unit derived from dicarboxylic acid in which each of Y^{1a} and Y^{1b} is represented by the formula (Y1) . These dicarboxylic acids may each be used singly, or two or more thereof may be used in combination.

Examples of the additional dicarboxylic acid component include, but are not particularly limited to, at least one dicarboxylic acid component selected from the group consisting of aliphatic dicarboxylic acid, alicyclic dicarboxylic acid, and aromatic dicarboxylic acid.

Examples of the aliphatic dicarboxylic acid include, but are not particularly limited to, alkanedicarboxylic acid (e.g., C₄₋₁₄ alkanedicarboxylic acid such as succinic acid, adipic acid, sebacic acid, and decanedicarboxylic acid, preferably C₆₋₁₂ alkanedicarboxylic acid), and unsaturated aliphatic dicarboxylic acid (e.g., C₂₋₁₀ alkene-dicarboxylic acid such as maleic acid, fumaric acid, and itaconic acid). The aliphatic dicarboxylic acid is preferably alkanedicarboxylic acid.

Examples of the alicyclic dicarboxylic acid component include, but are not particularly limited to, cycloalkanedicarboxylic acid (e.g., C₅₋₁₀ cycloalkanedicarboxylic acid such as 1,3-cyclohexanedicarboxylic acid and 1,4-cyclohexanedicarboxylic acid), di- or tri-cycloalkanedicarboxylic acid (e.g., decalindicarboxylic acid, norbornanedicarboxylic acid, adamantanedicarboxylic acid, and tricyclodecanedicarboxylic acid), cycloalkenedicarboxylic acid (e.g., C₅₋₁₀ cycloalkenedicarboxylic acid such as cyclohexenedicarboxylic acid), and di- or tri-cycloalkenedicarboxylic acid (e.g., norbornenedicarboxylic acid).

Examples of the aromatic dicarboxylic acid component include, but are not particularly limited to, monocyclic aromatic dicarboxylic acid [e.g., C₆₋₁₀ arenedicarboxylic acid such as phthalic acid, terephthalic acid, isophthalic acid, and alkylisophthalic acid (e.g., C₁₋₄ alkylisophthalic acid such as 4-methylisophthalic acid)], condensed polycyclic aromatic dicarboxylic acid [e.g., condensed polycyclic C₁₀₋₂₄ arene-dicarboxylic acid such as naphthalenedicarboxylic acid (e.g., 1,5-naphthalenedicarboxylic acid, 1,6-naphthalenedicarboxylic acid, 1,7-naphthalenedicarboxylic acid, 1,8-naphthalenedicarboxylic acid, and 2,6-naphthalenedicarboxylic acid), anthracenedicarboxylic acid, and phenanthrenedicarboxylic acid, preferably condensed polycyclic C₁₀₋₁₆ arene-dicarboxylic acid, further preferably condensed polycyclic C₁₀₋₁₄ arenedicarboxylic acid], arylarenedicarboxylic acid [e.g., C₆₋₁₀ aryl-C₆₋₁₀ arenedicarboxylic acid such as biphenyldicarboxylic acid (e.g., 2,2'-biphenyldicarboxylic acid, and 4,4'-biphenyldicarboxylic acid)], diarylalkanedicarboxylic acid [e.g., di-C₆₋₁₀ aryl-C₁₋₆ alkane-dicarboxylic acid such as diphenylalkanedicarboxylic acid (e.g., 4,4'-diphenylmethanedicarboxylic acid)], and diaryl ketone dicarboxylic acid [e.g., di-C₆₋₁₀ aryl ketone-dicarboxylic acid) such as diphenyl ketone dicarboxylic acid (e.g., 4.4'-diphenyl ketone dicarboxylic acid)].

The dicarboxylic acid component other than the ultraviolet absorber contained in the ultraviolet absorbing polyamide resin is not only free carboxylic acid but includes ester-forming derivatives of the dicarboxylic acid, for example, ester [e.g., alkyl ester [e.g., lower alkyl ester (e.g., C₁₋₄ alkyl ester, particularly, C₁₋₂ alkyl ester) such as methyl ester or ethyl ester]], acid halide (e.g., acid chloride), and acid anhydride. These dicarboxylic acid components may each be used singly, or two or more thereof may be used in combination.

### 1.2.3.2. Diamine

Examples of the diamine include, but are not particularly limited to, aliphatic diamine, alicyclic diamine, and aromatic diamine. These diamines may each be used singly, or two or more thereof may be combined.

Examples of the aliphatic diamine include alkanediamine (e.g., C₂₋₂₀ alkanediamine such as ethylenediamine, propylenediamine, 1,3-trimethylenediamine, 1,4-tetramethylenediamine, 1,5-pentamethylenediamine, 1,6-hexamethylenediamine, 1,7-heptamethylenediamine, 1,8-octamethylenediamine, 1,9-nonamethylenediamine, 1,10-decamethylenediamine, 1,11-undecamethylenediamine, 1,12-dodecamethylenediamine, 2-methyl-1,5-pentamethylenediamine, 2,2,4-trimethyl-1,6-hexamethylenediamine, 2,4,4-trimethyl-1,6-hexamethylenediamine, 2-methyl-1,8-octamethylenediamine, and 5-methyl-1,9-nonamethylenediamine, preferably C₂₋₁₂ alkanediamine, further preferably C₂₋₈ alkanediamine). These aliphatic diamines may each be used singly, or two or more thereof may be combined.

Examples of the alicyclic diamine include monocyclic cycloalkanediamine, bridged cyclic cycloalkanediamine, and isophoronediamine. These alicyclic diamines may each be used singly, or two or more thereof may be combined.

Examples of the monocyclic cycloalkanediamine can include: cycloalkanediamine such as diaminocyclohexane (1,4-diaminocyclohexane, etc.) and methylcyclohexanediamine (3-methyl-1,4-diaminocyclohexane, etc.); and bis(aminomethyl)cycloalkane such as bis(aminomethyl)cyclohexane (1,4-aminomethylcyclohexane, etc.) and bis(aminomethyl)methylcyclohexane. The monocyclic cycloalkanediamine is often C₄₋₁₀ cycloalkanediamine or bis(aminomethyl)-C₄₋₁₀ cycloalkane.

Examples of the bridged cyclic cycloalkanediamine can include: bi- or tri-cycloalkanediamine such as bicyclooctanediamine, bicyclononanediamine, tricyclododecanediamine, and norbornanediamine; and bis(aminomethyl)bi- or tri-cycloalkane such as bis(aminomethyl)bicyclooctane, bis(aminomethyl)bicyclononane, bis(aminomethyl)tricyclododecane, and bis(aminomethyl)norbornane. The bridged cyclic cycloalkanediamine is often C₆₋₁₄ bi- or tri-cycloalkanediamine or bis(aminomethyl) C₆₋₁₄ bi- or tri-cycloalkane.

Examples of the aromatic diamine include arenediamine (e.g., C₆₋₁₀ arenediamine such as m-phenylenediamine and p-phenylenediamine), amino alkyl-aminoarene [e.g., amino-C₁₋₄ alkyl-amino-C₆₋₁₀ arene such as α-(3-aminophenyl)ethylamine], and araliphatic diamine [e.g., di(amino alkyl) arene (e.g., di(amino-C₁₋₄ alkyl)C₆₋₁₀ arene) such as m-xylylenediamine and p-xylylenediamine]. These aromatic diamines may each be used singly, or two or more thereof may be combined.

Polymerizable components for the ultraviolet absorbing polyamide resin may be constituted by only the dicarboxylic acid component and the diamine component mentioned above, and may optionally include an aminocarboxylic acid component [aminocarboxylic acid (e.g., 6-aminocaproic acid and 12-aminododecanoic acid) and lactam (ε-caprolactam)].

The ratio of such an aminocarboxylic acid component may be, for example, 0.5 mol or less, preferably 0.3 mol or less, further preferably 0.2 mol or less, particularly, 0.1 mol or less, based on 1 mol of the dicarboxylic acid component.

### 1.2.3.3. Method for preparing polyamide resin

The ultraviolet absorbing polyamide resin can be produced by a common method. The polyamide resin can be produced, for example, by reacting (polymerizing or condensing) a dicarboxylic acid component with a diamine component. The polymerization method (production method) can be appropriately selected depending on the type of the dicarboxylic acid component used, etc. Examples thereof can include common methods, for example, a melt polymerization method (or melt polycondensation method; a method of polymerizing a dicarboxylic acid component with a diamine component under melt mixing), a solution polymerization method (or a solution polycondensation method), and an interfacial polymerization method (or an interfacial polycondensation method).

Specifically, in the melt polycondensation method, a salt prepared from the dicarboxylic acid component and the diamine component, or starting materials such as the dicarboxylic acid component and the diamine component are melted by heating to a temperature equal to or higher than the melting point thereof, and reaction can be performed while elimination components such as water or an alcohol are removed under reduced pressure from the reaction system. In the solution polycondensation method, reaction can be performed in a solvent while elimination components are removed by use of azeotropy, an acid acceptor such as tertiary amine, or a condensing agent such as a triphenyl phosphite/pyridine mixture system. In the interfacial polycondensation method, acid halide of dicarboxylic acid is dissolved in a nonpolar solvent and can be reacted with the diamine component at an interface with an aqueous solution of a compound, such as sodium hydroxide, which exhibits alkalinity.

In the melt polycondensation method, the salt of the dicarboxylic acid component and the diamine component can be obtained as precipitates by dissolving each of these components in a solvent and mixing the solutions. In such a case, the solvent is not particularly limited as long as the solvent is capable of dissolving the dicarboxylic acid component and the diamine component. Examples thereof include ethers (e.g., cyclic ethers such as tetrahydrofuran and 1,4-dioxane), ketones (e.g., chain ketones such as methyl ethyl ketone; and cyclic ketones such as cyclohexanone), halogen-containing solvents (e.g., halogenated hydrocarbons such as dichloromethane and chloroform), amides (e.g., N,N'-dimethylacetamide and N,N'-dimethylformamide), sulfoxides (e.g., dimethyl sulfoxide), and nitrogen-containing solvents (e.g., N-methylpyrrolidone). These solvents may each be used singly, or two or more thereof may be combined.

In the melt polycondensation method, for example, when any component (e.g., the diamine component) is volatile, the volatile component may be used in excess (e.g., at a proportion larger by 0.01 to 3% by mol or more than the theoretical amount). In order to prevent the diamine component or the like from volatilizing, the reaction may be performed under pressure at the initial stage of the reaction. The polymerization reaction may be performed under normal pressure or reduced pressure. It is preferred to reduce the pressure to around 150 Pa at the late stage of polymerization to promote the removal of elimination components such as water or an alcohol from the reaction system.

### 1.2.3.3. Physical properties of polyamide resin

The glass transition temperature of the ultraviolet absorbing polyamide resin can be selected from the range of 120°C or higher (e.g., 140°C or higher) and may be, for example, 150°C or higher (e.g., 160 to 400°C), preferably 165°C or higher (e.g., 165 to 370°C), further preferably 170°C or higher (e.g., 175 to 350°C), particularly, 180°C or higher (e.g., 190 to 320°C) or may be 200°C or higher (e.g., 200 to 350°C, preferably 210 to 300°C, further preferably 215 to 280°C). The glass transition temperature can be measured by a method described in Examples mentioned later.

The weight-average molecular weight of the ultraviolet absorbing polyamide resin is preferably 30000 to 200000, more preferably 35000 to 150000, further preferably 40000 to 110000. When the weight-average molecular weight falls within the range described above, the ultraviolet absorbing polyamide resin has a long molecular chain, mechanical characteristics such as elongation at break and flexibility tend to be more improved, and drawability tends to be more improved. In the present embodiment, the weight-average molecular weight can be measured by gel permeation chromatography (GPC) based on polystyrene. More specifically, the weight-average molecular weight can be measured by, for example, a method described in Examples mentioned later.

### 1.2.4. (Meth)acrylic resin

The (meth)acrylic resin is a polymerized form of (meth)acrylate and has the constituent unit F derived from the fluorene compound represented by the general formula (1) as the (meth)acrylate. Such a (meth)acrylic resin comprising the constituent unit F is also referred to as an "ultraviolet absorbing (meth)acrylic resin" for discrimination from other (meth)acrylic resins.

When the ultraviolet absorber represented by the general formula (1) of the present invention is di(meth)acrylate having reactivity, this di(meth)acrylate can be radical-polymerized to prepare an ultraviolet absorbing (meth)acrylic resin comprising an ultraviolet absorber component as a constituent unit.

The molar ratio of the constituent unit F contained in the ultraviolet absorbing (meth)acrylic resin is preferably 1 to 100% by mol, more preferably 5 to 80% by mol, further preferably 10 to 60% by mol, based on all radical-polymerizable compounds. When the content ratio of the ultraviolet absorber falls within the range described above, an ultraviolet absorbing property and ultraviolet absorption specificity at a specific wavelength tend to be more improved.

### 1.2.4.1. (Meth)acrylate monomer

All the (meth)acrylate units of the ultraviolet absorbing (meth)acrylic resin may be the constituent unit F, or the constituent unit F serving as (meth)acrylate may be combined with an additional radical polymerizable component. Examples of the constituent unit F serving as (meth)acrylate include, but are not particularly limited to, a constituent unit derived from (meth)acrylate in which each of Y^{1a} and Y^{1b} is represented by the formula (Y4). These (meth)acrylates may each be used singly, or two or more thereof may be used in combination.

Examples of the additional radical polymerizable component include, but are not particularly limited to, vinyl compounds, monofunctional (meth)acrylate, and polyfunctional (meth)acrylate.

Examples of the vinyl compound can include aromatic vinyl compounds such as styrene, α-methylstyrene, and vinyltoluene, fatty acid vinyl esters such as vinyl acetate, vinyl propionate, and vinyl pivalate, and N-vinyl compounds such as N-vinylpyrrolidone and N-vinylacetamide.

Examples of the monofunctional (meth)acrylate can include: alkyl (meth)acrylate [e.g., C₁₋₂₀ alkyl (meth)acrylate such as methyl (meth)acrylate, ethyl (meth)acrylate, and butyl (meth)acrylate], hydroxyalkyl (meth)acrylate, alkoxyalkyl (meth)acrylate, cyclohexyl (meth)acrylate, aryl (meth)acrylate (e.g., phenyl (meth)acrylate), aryloxyalkyl (meth)acrylate (e.g., phenoxyethyl (meth)acrylate), aralkyl (meth)acrylate (e.g., benzyl (meth)acrylate), aryloxy((poly)alkoxy)alkyl (meth)acrylate (e.g., phenoxyethoxyethyl (meth)acrylate), alkylaryloxy((poly)alkoxy)alkyl (meth)acrylate (e.g., nonylphenoxy(poly)ethoxyethyl (meth)acrylate), and arylaryl(oxy((poly)alkoxy)alkyl) (meth)acrylate (e.g., 2-(o-phenylphenoxy)ethyl (meth)acrylate and phenylphenoxy(poly)ethoxyethyl (meth)acrylate); (meth)acrylate having a sulfur atom [e.g., alkylthio (meth)acrylate (e.g., methylthio (meth)acrylate), arylthio (meth)acrylate (e.g., phenylthio (meth)acrylate), aralkylthio (meth)acrylate (e.g., benzylthio (meth)acrylate), and arylthioalkyl (meth)acrylate (e.g., phenylthioethyl (meth)acrylate)]; and N,N-dialkyl(meth)acrylamide (e.g., N,N-dimethyl(meth)acrylamide), N,N-dialkylaminoalkyl (meth)acrylate (e.g., N,N-dimethylaminoethyl (meth)acrylate), mono(meth)acrylate of bisphenols or alkylene oxide adducts thereof (e.g., mono(meth)acrylate of an ethylene oxide adduct of bisphenol A), and (meth)acrylate having a fluorene skeleton (e.g., 9-(meth)acryloyloxymethylfluorene).

Examples of the polyfunctional (meth)acrylate include difunctional (meth)acrylate [e.g., C₂₋₁₀ alkylene glycol di(meth)acrylate such as ethylene glycol di(meth)acrylate and butanediol di(meth)acrylate, polyalkylene glycol di(meth)acrylate such as diethylene glycol di(meth)acrylate, and di(meth)acrylate of bisphenol A (or alkylene oxide adducts thereof)], tri- or higher polyfunctional (meth)acrylate [e.g., tri- or tetra-(meth)acrylate of triol or tetraol such as trimethylolpropane tri(meth)acrylate, pentaerythritol tri(meth)acrylate, and pentaerythritol tetra(meth)acrylate], and oligo (meth)acrylate [urethane (meth)acrylate, epoxy (meth)acrylate, and polyester (meth)acrylate]. These monofunctional and polyfunctional (meth)acrylates can each be used singly, or two or more thereof can be used in combination.

### 1.2.4.2. Method for preparing (meth)acrylic resin

The ultraviolet absorbing (meth)acrylic resin can be prepared through the reaction of (meth)acrylate. The method for producing the (meth)acrylic resin is not particularly limited, and the (meth)acrylic resin may be prepared by a common method, for example, radical polymerization. A polymerization initiator, a photosensitizer, or the like may be used in polymerization reaction.

### 1.2.4.1. Polymerization initiator

A curable composition may comprise a polymerization initiator. This polymerization initiator may be a thermal polymerization initiator (thermal radical generator) or may be a photopolymerization initiator (photo radical generator).

Examples of the thermal polymerization initiator can include organic peroxide [dialkyl peroxides (e.g., di-tert-butyl peroxide), diacyl peroxides (e.g., lauroyl peroxide and benzoyl peroxide), peracids (or peracid esters) (e.g., tert-butyl hydroperoxide, cumene hydroperoxide, and tert-butyl peracetate), ketone peroxides, peroxycarbonate, and peroxyketals], and azo compounds [e.g., azonitrile compounds such as 2,2'-azobis(isobutyronitrile), azo amide compounds, and azo amidine compounds]. These thermal polymerization initiators can each be used singly, or two or more thereof can be used in combination.

Examples of the photopolymerization initiator can include benzoins (benzoin, benzoin alkyl ethers such as benzoin ethyl ether, etc.), acetophenones (acetophenone, 2-hydroxy-2-methyl-1-phenylpropan-1-one, etc.), aminoacetophenones {2-methyl-1-[4-(methylthio)phenyl]-2-morpholinoaminopropanone-1, etc.}, anthraquinones (anthraquinone, 2-methylanthraquinone, etc.), thioxanthones (2,4-dimethylthioxanthone, 2,4-diethylthioxanthone, 2-chlorothioxanthone, etc.), ketals (acetophenone dimethyl ketal, benzyl dimethyl ketal, etc.), benzophenones (benzophenone, etc.), and xanthones. These photopolymerization initiators may each be used singly, or two or more thereof may be used in combination.

The amount of the polymerization initiator (thermal or photopolymerization initiator) used may be on the order of 0.1 to 15 parts by mass, preferably 0.5 to 10 parts by mass (e.g., 1 to 8 parts by mass), further preferably 2 to 5 parts by mass, based on 100 parts by mass in total of radical polymerizable components.

The photopolymerization initiator may be combined with a photosensitizer. Examples of the photosensitizer include common photosensitizers such as tertiary amines {e.g., trialkylamine, trialkanolamine (triethanolamine, etc.), dialkylaminobenzoic acid alkyl ester such as ethyl N,N-dimethylaminobenzoate [ethyl p-(dimethylamino)benzoate, etc.] and amyl N,N-dimethylaminobenzoate [amyl p-(dimethylamino)benzoate, etc.], bis(dialkylamino)benzophenone such as 4,4-bis(diethylamino)benzophenone, and dialkylaminobenzophenone such as 4-(dimethylamino)benzophenone}. These photosensitizers may each be used singly, or two or more thereof may be combined.

The amount of the photosensitizer used may be on the order of 1 to 200 parts by mass, preferably 5 to 150 parts by mass, further preferably 10 to 100 parts by mass, based on 100 parts by mass of the polymerization initiator.

The ultraviolet absorber having a di(meth)acrylate structure is easily cured by the application of active energy (active energy beam) to produce an ultraviolet absorbing (meth)acrylic resin. Heat energy and/or light energy (ultraviolet ray, X ray, electron beam, etc.) is useful as the active energy.

In the case of using heat energy, the heating temperature may be on the order of, for example, 50 to 200°C, preferably 60 to 150°C, further preferably 70 to 120°C.

For light irradiation (e.g., ultraviolet irradiation), the dose of energy for light irradiation can be appropriately selected according to a purpose and may be on the order of, for example, 50 to 10000 mJ/cm², preferably 70 to 8000 mJ/cm², further preferably 100 to 5000 mJ/cm² (e.g., 500 to 3000 mJ/cm²).

Curing may be performed in air or in an inert gas (e.g., nitrogen or noble gas) atmosphere.

### 1.2.4.1. Physical properties of acrylic resin

The glass transition temperature of the ultraviolet absorbing (meth)acrylic resin can be selected from the range of -60°C to 300°C and may be on the order of preferably -40 to 250°C, further preferably -20 to 200°C. The glass transition temperature can be measured by a method described in Examples mentioned later.

The weight-average molecular weight of the ultraviolet absorbing (meth)acrylic resin can be selected from the range of 400 to 15000 (e.g., 600 to 12000) and may be on the order of 800 to 10000 (e.g., 1000 to 7500), further preferably 1200 to 6000 (e.g., 1500 to 5000). In the present embodiment, the weight-average molecular weight can be measured by gel permeation chromatography (GPC) based on polystyrene. More specifically, the weight-average molecular weight can be measured by, for example, a method described in Examples mentioned later.

### 1.3. Physical properties

The fluorene compound and the resin having the constituent unit F (hereinafter, also collectively referred to as an "ultraviolet absorber") according to the present embodiment may be in a crystalline form or may be in an amorphous form, depending on their chemical structures and production methods. When the ultraviolet absorber assumes a crystalline form, a plurality of crystal polymorphs may be present. Any form may be used for the purpose of the present invention, and a mixture thereof may also be accepted.

The half width of the ultraviolet absorber of the present embodiment is preferably 55 nm or less, more preferably 50 nm or less, further preferably 40 nm or less, still further preferably 30 nm or less. The lower limit of the half width is, for example, 3 nm or more, though a smaller half width is more preferred. When the half width is 55 nm or less, only ultraviolet ray in a specific range can be selectively absorbed and both an excellent UV absorbing property and a low staining property can be achieved. In this context, the half width refers to a wavelength band that exhibits a wavelength width of 1/2 or more of absorbance with reference to an absorption wavelength at which the absorbance is maximized.

The ultraviolet absorber of the present embodiment preferably has a maximum absorption wavelength within a wavelength range of 320 to 400 nm. When the maximum absorption wavelength falls within the range described above, ultraviolet ray in a long-wavelength region which is generally difficult to absorb can be suitably absorbed.

The molar absorption coefficient at 380 nm (A₃₈₀) is preferably 200 to 2000 L/(mol)·cm, more preferably 400 to 1500 L/(mol)·cm, further preferably 600 to 1000 L/ (mol)·cm.

The molar absorption coefficient at 400 nm (A₄₀₀) is preferably 0.5 to 50 L/(mol)·cm, more preferably 1.0 to 25 L/(mol)·cm, further preferably 2.0 to 10 L/(mol)·cm.

The molar absorption coefficient (Aₘₐₓ) of the maximum absorption wavelength is preferably 20000 to 100000 L/(mol)·cm, more preferably 30000 to 90000 L/(mol)·cm, further preferably 40000 to 80000 L/(mol)·cm.

When the molar absorption coefficient (A₃₈₀) or the molar absorption coefficient (A₄₀₀) falls within the range described above, an excellent UV absorbing property in a long-wavelength region is more improved and a staining property tends to be more reduced.

The ratio (A₃₈₀/A₄₀₀) of the molar absorption coefficient (A₃₈₀) to the molar absorption coefficient (A₄₀₀) is preferably 50 or more, more preferably 100 or more, further preferably 200 or more. The upper limit of the ratio (A₃₈₀/A₄₀₀) is, for example, 1000 or less, though a larger ratio is more preferred. When the ratio (A₃₈₀/A₄₀₀) is 50 or more, both an excellent UV absorbing property in a long-wavelength region and a low staining property can be achieved.

The melting point of the ultraviolet absorber of the present embodiment may be on the order of, for example, 100 to 250°C. A preferred range thereof is, in stages, 150 to 240°C, 175 to 230°C, or 180 to 225°C.

The 5% mass loss temperature of the ultraviolet absorber of the present embodiment may be on the order of, for example, 250 to 500°C. A preferred range thereof is, in stages, 350 to 480°C, 370 to 450°C, or 400 to 430°C.

### 2. Ultraviolet absorbing composition

The ultraviolet absorbing composition of the present embodiment comprises the ultraviolet absorber of the present embodiment and at least one or more compounds selected from a triazine ring-containing compound, a benzotriazole ring-containing compound, and a benzophenone ring-containing compound as a second ultraviolet absorber. The ultraviolet absorber of the present embodiment may be the fluorene compound described above or may be a resin such as an ultraviolet absorbing polyester resin, an ultraviolet absorbing polycarbonate resin, an ultraviolet absorbing polyamide resin, or an ultraviolet absorbing (meth)acrylic resin. The ultraviolet absorber of the present embodiment and the second ultraviolet absorber thus mixed at an arbitrary ratio are capable of absorbing ultraviolet ray in a wider range of wavelengths.

The second ultraviolet absorber can be freely selected according to the absorption wavelength of interest, compatibility with a resin mentioned later, etc. As one example, a triazine ring-containing compound is preferably selected for the purpose of absorbing ultraviolet ray in a wide range of wavelengths in a UV-A region. A benzotriazole ring-containing compound is preferably selected for the purpose of being compatible with a general-purpose resin. A benzophenone ring-containing compound is preferably selected for the purpose of being compatible with a low polar resin. The ratio of the second ultraviolet absorber contained in the ultraviolet absorbing composition of the present embodiment can be freely selected according to a purpose and specifically, is preferably 0.01 to 50% by mass.

### 3. Ultraviolet absorbing resin composition

The ultraviolet absorbing resin composition of the present embodiment comprises the ultraviolet absorber of the present embodiment and an arbitrary resin. The ultraviolet absorber of the present embodiment may be the fluorene compound described above or may be a resin such as an ultraviolet absorbing polyester resin, an ultraviolet absorbing polycarbonate resin, an ultraviolet absorbing polyamide resin, or an ultraviolet absorbing (meth)acrylic resin.

The content of the ultraviolet absorber of the present embodiment is preferably 0.1 to 10% by mass, more preferably 0.5 to 8.0% by mass, further preferably 1.0 to 5.0% by mass, based on the total amount of the ultraviolet absorbing resin composition. When the amount of the ultraviolet absorber used is 0.1% by mass or more, ultraviolet absorption performance tends to be more improved. On the other hand, when the amount of the ultraviolet absorber used is 10% by mass or less, physical properties such as rigidity and toughness tend to be more improved.

Examples of the arbitrary resin include thermoplastic resins and thermosetting resins.

The ultraviolet absorbing resin composition can be processed, for use, into an arbitrary form such as a plate, sheet, or film shape, if necessary. The resin composition for use in the ultraviolet absorbing resin composition is preferably a thermoplastic resin because of its excellent moldability and processability.

### 3.1. Thermoplastic resin

The thermoplastic resin is not particularly limited as long as the thermoplastic resin is compatible with the ultraviolet absorber of the present embodiment. Examples thereof include: polyolefin-based resins such as chain olefin-based resins and cyclic olefin-based resins (or cycloolefin-based resins); (meth)acrylic resins; styrene-based resins such as polystyrene, AS resins, and ABS resins; polyester-based resins such as polyalkylene arylate resins, polyarylate resins, polyethylene terephthalate, and polycarbonate resins; polyamide-based resins; cellulose-based resins such as triacetylcellulose; vinyl resins such as polyvinyl chloride, polyvinyl acetate, and polyvinyl alcohol; and thermoplastic polyurethane resins.

### 3.2. Thermosetting resin

The thermosetting resin is not particularly limited as long as the thermosetting resin is compatible with the ultraviolet absorber of the present embodiment. Examples thereof include: phenol resins; epoxy resins; melamine resins; urine resins; unsaturated polyester resins; alkyd resins; thermosetting polyurethane resins; and thermosetting polyimide resins.

### 3.3. Method for preparing composition

A known approach can be used as an approach of melt-kneading the ultraviolet absorbing resin composition. Examples thereof include single-screw extruders, twin-screw extruders, Henschel mixers, Banbury mixers, tandem mixers, and co-kneaders. Usually, production by melt kneading in a twin-screw extruder is preferred because uniform and complete mixing can be attained.

### 3.4. Other components

The ultraviolet absorbing resin composition may optionally comprise various additives [e.g., a filler or a reinforcing agent, a colorant (dye or pigment), a conductive agent, a flame retardant, a plasticizer, a lubricant, a stabilizer (antioxidant, ultraviolet absorber, a heat stabilizer, etc.), a mold release agent, an antistatic agent, a dispersant, a fluidity adjuster, a leveling agent, an antifoaming agent, a surface modifier, a stress lowering agent (silicone oil, silicone rubber, various plastic powders, various engineering plastic powders, etc.), a heat resistance improving agent (sulfur compound, polysilane, etc.), and a carbon material]. These additives may each be used singly, or two or more thereof may be combined.

### 4. Ultraviolet absorbing coating liquid

The ultraviolet absorbing coating liquid of the present embodiment comprises the ultraviolet absorber of the present embodiment, the ultraviolet absorbing composition of the present embodiment, or the ultraviolet absorbing resin composition of the present embodiment, and a solvent. Thus, the ultraviolet absorber, the ultraviolet absorbing composition, or the ultraviolet absorbing resin composition of the present embodiment can be dissolved in an arbitrary solvent and thereby prepared into an ultraviolet absorbing coating liquid.

Examples of the solvent that can be used in the ultraviolet absorbing coating liquid include: aromatic solvents such as benzene and toluene; ketone-based solvents such as diacetone alcohol, acetone, cyclohexanone, cyclopentanone, methyl ethyl ketone, and methyl isopropyl ketone; cycloalkane-based solvents such as cyclohexane, ethylcyclohexane, and 1,2-dimethylcyclohexane; halogen-containing solvents such as methylene chloride and chloroform; and ether-based solvents such as tetrahydrofuran and dioxane. The concentration of the resin in the coating liquid can be 1% by mass to 50% by mass from the viewpoint of obtaining a viscosity suitable for coating. One type of solvent may be used singly, or two or more types of solvents may be used in combination at an arbitrary ratio.

The ultraviolet absorbing coating liquid may be supplemented, if necessary, with an additive such as an antifoaming agent, a leveling agent, an antioxidant, a light stabilizer, a lubricant, a flame retardant, or an antistatic agent. These additives may each be used singly, or two or more thereof may be combined.

### 5. Ultraviolet absorbing coated resin sheet and ultraviolet absorbing coated glass

The ultraviolet absorbing coated resin sheet of the present embodiment has a resin sheet and an ultraviolet shielding layer, and the ultraviolet shielding layer comprises the ultraviolet absorber, the ultraviolet absorbing composition, or the ultraviolet absorbing resin composition of the present embodiment. The ultraviolet absorbing coated glass of the present embodiment has glass and an ultraviolet shielding layer, and the ultraviolet shielding layer comprises the ultraviolet absorber, the ultraviolet absorbing composition, or the ultraviolet absorbing resin composition of the present embodiment.

The resin sheet is not particularly limited as long as the resin sheet can be coated with the ultraviolet absorber, the ultraviolet absorbing composition, or the ultraviolet absorbing resin composition of the present embodiment. Examples thereof include sheets made of polycarbonate-based resins, polyamide-based resins, polyvinyl alcohol-based resins, cellulose ester-based resins such as triacetylcellulose films and cellulose acetate propionate films, polyester-based resins such as polyethylene terephthalate and polyethylene naphthalate, polyarylate-based resins, polyimide-based resins, cycloolefin-based resins, polysulfone-based resins, polyethersulfone-based resins, or polyolefin-based resins such as polyethylene and polypropylene.

The glass is not particularly limited as long as the glass can be coated with the ultraviolet absorbing coating liquid. Examples thereof include float glass, reinforced glass, semi-reinforced glass, chemically reinforced glass, green glass, and quarts glass.

The method for coating the ultraviolet shielding layer is not limited. Coating with the ultraviolet absorber, the ultraviolet absorbing composition, or the ultraviolet absorbing resin composition may be performed by coating the resin sheet or the glass with the ultraviolet absorbing coating liquid.

Examples of the coating method include a curtain coating method, an extrusion coating method, a roll coating method, a spin coating method, a dip coating method, a bar coating method, a spray coating method, a slide coating method, a printing coating method, a gravure coating method, a die coating method, a gap coating method, and a dipping method.

The method for drying the ultraviolet absorbing coating liquid is not limited. For example, a drying method such as drying by heating or drying under reduced pressure may be used.

### 6. Ultraviolet absorbing film and ultraviolet absorbing molded article

The ultraviolet absorbing film of the present embodiment comprises the ultraviolet absorber, the ultraviolet absorbing composition, or the ultraviolet absorbing resin composition of the present embodiment. The ultraviolet absorbing molded article of the present embodiment comprises the ultraviolet absorber, the ultraviolet absorbing composition, or the ultraviolet absorbing resin composition of the present embodiment.

The ultraviolet absorbing molded article can be prepared by an arbitrary molding method. Examples of the shape of the molded article include, but are not particularly limited to, two-dimensional structures such as film, sheet, and plate shapes, and three-dimensional structures such as rod, tube, and hollow shapes. The ultraviolet absorbing molded article of the present invention is excellent in optical characteristics, mechanical characteristics, and high heat resistance and is therefore particularly suitable for optical members such as optical films, optical lenses, and optical sheets.

The ultraviolet absorbing molded article may optionally comprise various additives [e.g., a filler or a reinforcing agent, a colorant (dye or pigment), a conductive agent, a flame retardant, a plasticizer, a lubricant, a stabilizer (antioxidant, ultraviolet absorber, a heat stabilizer, etc.), a mold release agent, an antistatic agent, a dispersant, a fluidity adjuster, a leveling agent, an antifoaming agent, a surface modifier, a stress lowering agent (silicone oil, silicone rubber, various plastic powders, various engineering plastic powders, etc.), a heat resistance improving agent (sulfur compound, polysilane, etc.), and a carbon material]. These additives may each be used singly, or two or more thereof may be combined.

The ultraviolet absorbing molded article can be produced by use of, for example, an injection molding method, an injection compression molding method, an extrusion molding method, a transfer molding method, a blow molding method, a press molding method, or a casting molding method.

The resin serving as the ultraviolet absorber of the present embodiment or the ultraviolet absorbing resin composition may be molded into a film shape to prepare an ultraviolet absorbing film. In this case, the film may be a single-layer film consisting of only the resin serving as the ultraviolet absorber or the ultraviolet absorbing resin composition.

Examples of the method for forming the single-layer film include, but are not particularly limited to, a solution casting method, an inflation method, melt extrusion such as a T die method, and a calendering method. Among them, a melt extrusion method such as a T die method is preferred from the viewpoint that not only is productivity excellent but reduction in optical characteristics due to a residual solvent can be prevented. A solution casting method is preferred from the viewpoint that not only can a film thickness be controlled highly accurately but a thin single-layer film can be formed.

In the solution casting method, a base material is coated with a coating liquid of the ultraviolet absorbing polyester resin, the ultraviolet absorbing polycarbonate resin, the ultraviolet absorbing polyamide resin, the ultraviolet absorbing (meth)acrylic resin, or the ultraviolet absorbing resin composition dissolved in a solvent, and the solvent can be removed by drying, followed by the peeling of the base material to obtain a single-layer film. The base material may be a resin film, a resin sheet, or glass and is preferably a resin film from the viewpoint of productivity, further preferably a resin film subjected to mold release treatment so as to attain easy peeling.

The coating method using the coating liquid is not limited. Examples of the coating method include a curtain coating method, an extrusion coating method, a roll coating method, a spin coating method, a dip coating method, a bar coating method, a spray coating method, a slide coating method, a printing coating method, a gravure coating method, a die coating method, a gap coating method, and a dipping method.

The method for drying the coating liquid is not limited. For example, a drying method such as drying by heating or drying under reduced pressure may be used.

In the melt extrusion method such as a T die method, molding conditions are adjusted according to the glass transition temperature or melting point of the ultraviolet absorbing polyester resin, the ultraviolet absorbing polycarbonate resin, the ultraviolet absorbing polyamide resin, the ultraviolet absorbing (meth)acrylic resin, or the ultraviolet absorbing resin composition, and molding can be performed by a common method. As for specific molding conditions, the melting temperature of the resin to be extruded is preferably Tg + 80°C or higher, more preferably Tg + 100°C or higher, and preferably Tg + 180°C or lower, more preferably Tg + 150°C or lower. The melting temperature refers to the melting temperature of the resin in an extruder having a T die, for example, in the T die method. When the melting temperature of the resin to be extruded is equal to or higher than the lower limit value of the range described above, the resin can have sufficiently high fluidity and favorable moldability. When the melting temperature is equal to or lower than the upper limit value, the deterioration of the resin can be suppressed.

The ultraviolet absorbing film of the present invention may not only be a single-layer film but be a laminated film in which the ultraviolet absorbing film is laminated with another film.

Examples of the molding method for the laminated film include, but are not particularly limited to, a coextrusion molding method, a solution casting method, and an extrusion laminate molding method. Among them, a coextrusion molding method is preferred from the viewpoint that not only is productivity excellent but reduction in optical characteristics or mechanical characteristics due to a residual solvent can be prevented.

Examples of a coextrusion T die method, which is a coextrusion molding method, include a feed block system and a multi-manifold system. A multi-manifold system is particularly preferred because variations in thickness among layers can be reduced.

The ultraviolet absorbing film of the present invention may be an undrawn film or may be a drawn film from the viewpoint of mechanical characteristics. Drawing treatment is effective as a film thinning approach. When an undrawn film has a thickness of roughly 50 µm or larger, the film thickness can be controlled by sandwich between nip rolls after casting from a T die. Thus, film thickness accuracy is improved. A thin film with good film thickness accuracy can be obtained by selecting drawing conditions under which uniform drawing stress is obtained, and performing drawing treatment.

Molding by drawing can be performed while the ultraviolet absorbing film formed by any of the methods described above is heated to an appropriate temperature between the melting point and the glass transition point. The drawing may be biaxial drawing or uniaxial drawing and can be selected according to subsequent embodiments. For use as a polarizer protective film mentioned later, biaxial drawing is preferred because less retardation ascribable to drawing is exerted. The biaxial drawing can draw the film in two directions, i.e., longitudinally and transversely, and in-plane retardation Ro can be canceled longitudinally and transversely and becomes a value close to zero. On the other hand, uniaxial drawing is preferred for use as a film that appropriately exhibits phase difference according to need, such as a 1/4λ phase difference film.

### 7. Polarizer protective film

The ultraviolet absorbing film of the present embodiment can be used as a polarizer protective film.

The total thickness of the polarizer protective film is preferably 5 to 90 µm, more preferably 10 to 80 µm, further preferably 20 to 50 µm. When the total thickness falls within the range described above, the polarizer protective film can be more suitably used.

The spectral transmittance of the polarizer protective film of the present embodiment at 350 nm is preferably 5% or less, more preferably 3% or less, further preferably 1% or less. When the spectral transmittance at a wavelength of 350 nm falls within the range described above, the polarizer protective film can prevent a polarizer from being deteriorated due to ultraviolet ray contained in outside light, and can be more suitably used.

The b* value in the L*a*b* color space (CIELAB) of the polarizer protective film of the present embodiment is preferably 0.8 or less, more preferably 0.6 or less, further preferably 0.5 or less. The b* value is preferably -0.8 or more, more preferably -0.6 or more, further preferably -0.5 or more. When the b* value falls within the range described above, the resulting polarizer protective film can be excellent in viewability. The b* value mentioned above is a value calculated in accordance with JIS Z8781-4: 2013.

The a* value is preferably 0.3 or less, more preferably 0.2 or less, further preferably 0.1 or less. The a* value is preferably -0.4 or more, more preferably -0.3 or more, further preferably -0.2 or more.

The L* value may be preferably 100 or less, may be 99 or less, or may be 98 or less. The L* value is preferably 90 or more, 92 or more, 94 or more.

The spectral transmittance of the polarizer protective film of the present embodiment at 380 nm is preferably 8% or less, more preferably 5% or less, further preferably 1% or less. When the spectral transmittance at a wavelength of 380 nm falls within the range described above, the polarizer protective film can prevent a polarizer from being deteriorated due to ultraviolet ray contained in outside light, and can be more suitably used.

### 8. Polarizing plate

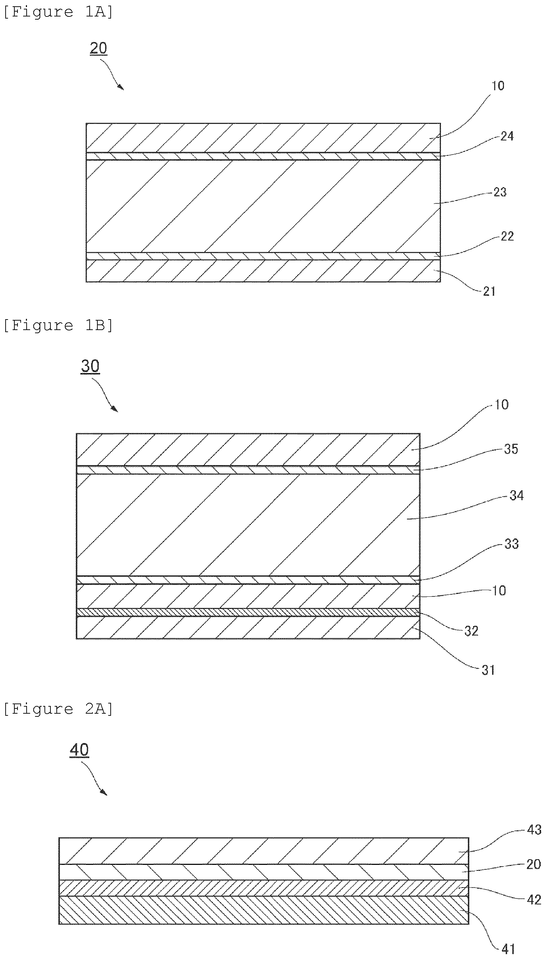

The polarizing plate of the present embodiment has the polarizer protective film and may optionally further have another layer. Next, the polarizing plate of the present embodiment will be described with reference to Figures 1A and 1B. This polarizing plate comprises the polarizer protective film of the present embodiment. Each of Figures 1A and 1B is a cross-sectional view schematically illustrating one form of the polarizing plate.

Polarizing plate 20 shown in Figure 1A is a laminate of phase difference film 21, polarizer 23, and polarizer protective film 10 in this order. As shown in this drawing, adhesive layer 22 may be disposed between the phase difference film 21 and the polarizer 23, and adhesive layer 24 may be disposed between the polarizer 23 and the polarizer protective film 10.

Polarizing plate 30 shown in Figure 1B is a laminate of phase difference film 31, polarizer protective film 10, polarizer 34, and polarizer protective film 10 in this order. Adhesive layer or pressure-sensitive adhesive layer 32 may be disposed between the phase difference film 31 and the polarizer protective film 10, and adhesive layer 33 or 35 may be disposed between the polarizer 34 and the polarizer protective film 10.

The polarizer protective film 10 may be subjected to corona treatment, plasma treatment, or surface modification treatment with an aqueous solution of a strong base such as sodium hydroxide or potassium hydroxide in order to improve adhesion to the polarizer 23 or 34. Such surface modification treatment may be performed after a film formation step or may be performed after a drawing step.

The polarizer 23 or 34 is not particularly limited as long as the polarizer is a conventionally known one. Examples thereof include: films obtained by subjecting hydrophilic polymer films such as polyvinyl alcohol films, partially formalized polyvinyl alcohol films, or ethylene-vinyl acetate copolymer-based partially saponified films to staining treatment with iodine or a dichroic substance such as a dichroic dye and drawing treatment; and polyene-based oriented films such as dehydration treatment products of polyvinyl alcohol and dehydrochlorination treatment products of polyvinyl chloride. Other examples thereof include polarizers obtained by staining polyvinyl alcohol films with iodine, followed by uniaxial drawing.

The polarizer protective film described above can be used as the polarizer protective film 10. The polarizer protective film 10 and the polarizer 23 or 34 formed from a polyvinyl alcohol-based resin or the like may be laminated through an ultraviolet-curable adhesive (adhesive layer 24, 33, or 35).

### 9. Image display apparatus

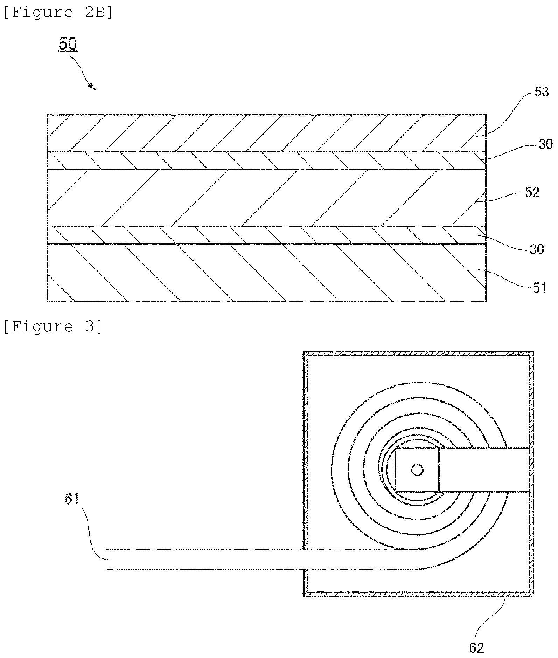

The image display apparatus of the present embodiment has the polarizing plate. Next, the image display apparatus of the present embodiment will be described with reference to Figures 2A and 2B. The image display apparatus of the present embodiment is not particularly limited as long as the image display apparatus comprises the polarizing plate. Examples thereof include organic electroluminescence (EL) display apparatuses and liquid crystal display apparatuses. The image display apparatus is not only an apparatus that is distributed as a final product in itself to the market but may be a portion of an information processing apparatus mentioned later, for example, a smartphone. Figure 2A is a cross-sectional view schematically illustrating an organic EL display apparatus according to one aspect of the present embodiment. Figure 2B is a cross-sectional view schematically illustrating a liquid crystal display apparatus according to one aspect of the present embodiment.

As shown in Figure 2A, organic EL display apparatus 40 has organic EL display panel 41, polarizing plate 20 comprising the polarizer protective film 10 of the present embodiment, and front panel 43 in this order. In the organic EL display apparatus 40, use of polarizing plate 20 having polarizer protective film 10 can prevent the polarizing plate 20 from being deteriorated by ultraviolet ray or moisture permeation and achieves excellent mechanical strength against flex and a thinner polarizing plate.

The organic EL display apparatus 40 may optionally have an additional configuration such as touch sensor 42. The organic EL display apparatus 40 equipped with the touch sensor 42 functions as an information input interface, in addition to a function as a display apparatus. The respective layers constituting the organic EL display apparatus 40 may be joined to each other using a pressure-sensitive adhesive or an adhesive.

As shown in Figure 2B, liquid crystal display apparatus 50 has light source 51, polarizing plate 30, liquid crystal panel 52, polarizing plate 30, and front panel 53 in this order. The light source 51 may be a direct-lit system in which light sources are evenly arranged immediately beneath the liquid crystal panel, or may be an edge-lit system having a reflector and a light guide panel. Although Figure 2B shows the front panel 53, the liquid crystal display apparatus 50 may have no front panel 53. The liquid crystal display apparatus 50 may further have a touch sensor (not shown).

The screen of the image display apparatus is not limited by a rectangular shape and may have a round, oval, or polygonal (e.g., triangular or pentagonal) shape. The image display apparatus can further have flexibility, and its shape may be changed such that the image display apparatus is arched, bent, wound, or folded. For example, as shown in Figure 3, the image display apparatus includes a rollable display that can be used such that a roll of image display apparatus 61 housed in image display apparatus housing 62 is taken out.

### 10. Transport equipment

The ultraviolet absorber, the ultraviolet absorbing composition, or the ultraviolet absorbing resin composition, the ultraviolet absorbing coating liquid, the ultraviolet absorbing coated resin sheet, the ultraviolet absorbing coated glass, the ultraviolet absorbing molded article, the ultraviolet absorbing film, the polarizer protective film, the polarizing plate, or the image display apparatus of the present embodiment can be suitably used as a member, for example, for transport equipment such as an automobile, a ship, or an aircraft.

### 11. Building member

The ultraviolet absorber, the ultraviolet absorbing composition, or the ultraviolet absorbing resin composition, the ultraviolet absorbing coating liquid, the ultraviolet absorbing coated resin sheet, the ultraviolet absorbing coated glass, the ultraviolet absorbing molded article, the ultraviolet absorbing film, the polarizer protective film, the polarizing plate, or the image display apparatus of the present embodiment can be used as a building member including a structural material for pillars, beams, or the like, a roof material, a wall material, a floor material, and joinery for doors, windows, or the like, and can be particularly suitably used as a window member for the purpose of absorbing ultraviolet ray invading for lighting.

### Example 1

Hereinafter, the present invention will be described in more detail with reference to Examples. However, the present invention is not limited by these Examples. Evaluation methods and starting materials are given below.

### 1. Evaluation method

### 1.1. Glass transition temperature (Tg)

A differential scanning calorimeter ("DSC 6220" manufactured by Seiko Instruments Inc.) was used. A sample was placed in an aluminum pan, and Tg was measured in the range of 30°C to 200°C in accordance with JIS K 7121.

### 1.2. Weight-average molecular weight

Gel permeation chromatography (manufactured by Tosoh Corp., "HLC-8120GPC") was used. A sample was dissolved in chloroform, and polystyrene-based weight-average molecular weight Mw of each resin was measured.

### 1.3. 5% mass loss temperature

The temperature at which the mass of a sample was decreased by 5% by mass was measured in a nitrogen atmosphere under conditions involving a temperature elevation rate of 10°C/min using a thermogravimeter ("TGA 4000" manufactured by Perkin Elmer, Inc.).

### 1.4. Film thickness

A thickness gauge ("Micrometer" manufactured by Mitsutoyo Corp.) was used. Three equally spaced points between chucks were measured in the longitudinal direction of a film, and an average value thereof was calculated.

### 1.5. Molar absorption coefficient, maximum absorption wavelength, and half width

A tetrahydrofuran solution having a concentration suitable for measurement mentioned later was prepared as to each sample, and ultraviolet to visible spectra were measured in a 1 cm quartz cell using an ultraviolet-visible spectrophotometer ("V-650" manufactured by JASCO Corp.). In this context, the concentration suitable for measurement is in a range where absorbance indicates 0.1 to 2.0. A measurement concentration in this range and absorbance were plotted as to three or more points, and a molar absorption coefficient was calculated from the slope. A maximum absorption wavelength and a half width were calculated from the obtained spectrum chart.

### 1.4. Transmittance

Transmittance was measured as to each film at each of 350 nm and 380 nm using an ultraviolet-visible spectrophotometer ("V-650" manufactured by JASCO Corp.) in accordance with the specification of JIS K7361: 1997.

### 1.5. L*a*b* color space

Each film was measured at each of 350 nm and 380 nm using an ultraviolet-visible spectrophotometer ("V-650" manufactured by JASCO Corp.) in accordance with the specification of JIS Z8729. The L* value represents lightness, and the a* value and the b* value represent hues, respectively.

### 2. Fluorene compound

### 2.1. Example 1: Preparation of DNFDP-m

200 ml of 1,4-dioxane and 54.7 g (0.17 mol) of 2,7-dibromo-9H-fluorene were placed in a reactor, and the fluorene was dissolved by stirring. Then, 3.0 ml of a solution containing 40% by mass of trimethylbenzylammonium hydroxide in methanol (Triton B40 manufactured by Tokyo Chemical Industry Co., Ltd.) was added dropwise thereto in a state cooled to 10°C, and the mixture was stirred for 30 minutes. Next, 37.9 g (0.44 mol) of methyl acrylate was added thereto, and the mixture was stirred for approximately 3 hours. After the completion of the reaction, the reaction mixture was washed by the addition of 200 ml of toluene and 50 ml of 0.5 N hydrochloric acid. The aqueous layer was removed, and the organic layer was then washed three times with 30 ml of distilled water. The solvent was distilled off to obtain 9,9-bis(2-methoxycarbonylethyl)-2,7-dibromofluorene (DBrFDP-m).

Subsequently, a reactor was charged with 192.3 g (0.39 mol) of the obtained DBrFDP-m, 200 g (1.2 mol) of 2-naphthylboronic acid, 4.3 L of dimethoxyethane, and 1 L of a 2 M aqueous sodium carbonate solution, and 22.4 g (19.4 mmol) of tetrakis(triphenylphosphine)palladium(0) [or Pd(PPh₃)₄] was added thereto under nitrogen stream. The mixture was heated to reflux at an internal temperature of 71 to 78°C for 5 hours and reacted. After cooling to room temperature, 2.0 L of toluene and 500 mL of ion exchange water were added thereto, followed by extraction five times for separation into organic and aqueous layers and washing. The color of the organic layer was changed from dark orange to brown. Insoluble matter was filtered and concentrated to obtain crude brown crystals. The obtained crude crystals were dissolved by heating in a mixed solution of 1.5 kg of ethyl acetate and 300 g of isopropyl alcohol (IPA). Then, the solution was cooled to 10°C or lower in ice water and stirred for 1 hour to deposit crystals. The deposited crystals were filtered and then dried under reduced pressure to obtain 130 g of gray-brown crystals. The obtained gray-brown crystals were purified by column chromatography (silica gel support, developing solvent chloroform:ethyl acetate (volume ratio) = 4:1), then recrystallized with methanol, and dried under reduced pressure to obtain 9,9-bis(2-methoxycarbonylethyl)-2,7-di(2-naphthyl)fluorene (DNFDP-m). Results of ¹H-NMR and FD-MS are shown below.
¹H-NMR (CDCl₃, 300 MHz): δ (ppm) 1.7 (t, 4H), 2.6 (t, 4H), 3.4 (s, 6H), 7.5 (m, 4H), 7.7-8.0 (m, 14H), 8.1 (s, 2H)
FD-MS: m/z590 (M+)

### 2.2. Example 2: Preparation of DNFPO

In a nitrogen atmosphere, DNFDP-m (1.2 kg, 2.03 mol) obtained as described above and tetrahydrofuran (THF, 9.78 kg) were added to a 20 L separable flask and cooled to 10°C or lower in an ice water bath. To the obtained solution, sodium borohydride (230 g, 6.08 mol) was added in small portions over 13 minutes, subsequently a boron trifluoride-ether complex (856 g, 6.09 mol) was added dropwise over 45 minutes, then the temperature was elevated to room temperature, and the mixture was stirred for 4 hours. After cooling in an ice water bath, acetone (1.0 kg, 17.2 mol) was added thereto over 1 hour, and the solvent was then distilled off by concentration under reduced pressure. Then, 12 kg of chloroform, 1.0 kg of ion exchange water, and 3.0 kg of ice were added to the residue, and an aqueous layer was removed. An operation of adding 4.0 kg of ion exchange water and removing an aqueous layer was further performed twice (washing twice with ion exchange water) to isolate only an organic layer. The obtained organic layer was concentrated under reduced pressure to obtain 9,9-bis(3-hydroxypropyl)-2,7-di(2-naphthyl)fluorene (DNFPO). Results of ¹H-NMR and FD-MS are shown below.

¹H-NMR (DMSO-d₆, 300 MHz): δ (ppm) 0.9 (m, 4H), 2.2 (m, 4H), 3.2 (t, 4H), 4.2 (t, 2H), 7.5-8.4 (m, 20H).
FD-MS: m/z 535 (M+)

### 2.3. Example 3: Preparation Example of DNFPA

A 500 mL three-neck flask equipped with a Dean-Stark apparatus was charged with 30.0 g (0.06 mol) of DNFPO obtained as described above, 10.5 g (0.15 mol) of acrylic acid, 55 g of toluene, and 0.12 g (1.0 mmol) of 2-methoxyphenol. The system was purged with nitrogen, and the temperature was elevated to 95°C so that the components were homogenized. Then, 1.33 g (7.0 mmol) of p-toluenesulfonic acid monohydrate was added thereto, and the system was purged with nitrogen again, followed by reflux dehydration for 4 hours. The reaction temperature was 110 to 115°C.

The obtained solution was washed (internal temperature: 60 to 70°C) with 195 g of toluene and 20 g of 20% by mass of saline and then neutralized (internal temperature: 60 to 70°C) with 20 g of 10% caustic soda water (aqueous solution of 10% by mass of sodium hydroxide) and 20 g of 20% by mass of saline, and an aqueous layer was confirmed to have pH 10 or higher. 2-Methoxyphenol was added at 500 ppm by mass to the whole organic layer so that the solution was homogenized. The solution was washed (internal temperature: 60 to 70°C) twice with 20 g of 20% by mass of saline and twice with 20 g of ion exchange water, and an aqueous layer was confirmed to have pH 7. Then, 6 g of active carbon (FP-6 manufactured by Mizusawa Industrial Chemicals, Ltd.) was added to the organic layer, and the mixture was stirred at room temperature for 1 hour, filtered through celite, then concentrated, and dried under reduced pressure overnight at 100°C to obtain 9,9-bis(3-acryloyloxypropyl)-2,7-di(2-naphthyl)fluorene.

### 2.4. Example 4: Preparation Example of DPFDP-m

A reactor was charged with 192.3 g (0.39 mol) of DBrFDP-m obtained as described above, 150 g (1.2 mol) of phenylboronic acid, 4.3 L of dimethoxyethane, and 1 L of a 2 M aqueous sodium carbonate solution, and 22.4 g (19.4 mmol) of tetrakis(triphenylphosphine)palladium(0) [or Pd(PPh₃)₄] was added thereto under nitrogen stream. The mixture was heated to reflux at an internal temperature of 71 to 78°C for 5 hours and reacted. After cooling to room temperature, 2.0 L of toluene and 500 mL of ion exchange water were added thereto, followed by extraction five times for separation into organic and aqueous layers and washing. The color of the organic layer was changed from dark orange to brown. Insoluble matter was filtered and concentrated to obtain crude brown crystals. The obtained crude crystals were dissolved by heating in a mixed solution of 1.5 kg of ethyl acetate and 300 g of isopropyl alcohol (IPA). Then, the solution was cooled to 10°C or lower in ice water and stirred for 1 hour to deposit crystals. The deposited crystals were filtered and then dried under reduced pressure to obtain 110 g of gray-brown crystals. The obtained gray-brown crystals were purified by column chromatography (silica gel support, developing solvent chloroform:ethyl acetate (volume ratio) = 4:1), then recrystallized with methanol, and dried under reduced pressure to obtain 9,9-bis(2-methoxycarbonylethyl)-2,7-diphenylfluorene (DPFDP-m).

### 2.5. Example 5: Preparation of BNEF

A 1 L separable flask was charged with 45 g (0.25 mol) of 9-fluorenone (manufactured by Osaka Gas Chemicals Co., Ltd.), 188 g (1 mol) of ethylene glycol mono(2-naphthyl) ether, and 1 g of 3-mercaptopropionic acid, which were then completely dissolved by warming to 60°C. Then, 54 g of sulfuric acid was gradually added thereto, and the mixture was stirred for 5 hours with 60°C maintained. As a result, it was able to be confirmed that the degree of conversion of 9-fluorenone was 99% or more in HPLC (high-performance liquid chromatography). The obtained reaction solution was neutralized by the addition of 48% caustic soda water. Then, 400 g of xylene was added thereto, and the mixture was washed several times with distilled water and cooled to deposit crystals. The crystals were further filtered and dried to obtain 9,9-bis[6-(2-hydroxyethoxy)-2-naphthyl]fluorene (BNEF) .

### 2.6. Example 6: BPEF

In Example 6, BPEF: 9,9-bis[4-(2-hydroxyethoxy)phenyl]fluorene manufactured by Osaka Gas Chemicals Co., Ltd. was used as a fluorene compound.

### 2.7. Example 7: Preparation of FDP-m

200 ml of 1,4-dioxane and 33.2 g (0.2 mol) of fluorene were placed in a reactor, and the fluorene was dissolved by stirring. Then, 3.0 ml of a solution containing 40% by mass of trimethylbenzylammonium hydroxide in methanol (Triton B40 manufactured by Tokyo Chemical Industry Co., Ltd.) was added dropwise thereto in a state cooled to 10°C, and the mixture was stirred for 30 minutes. Next, 37.9 g (0.44 mol) of methyl acrylate was added thereto, and the mixture was stirred for approximately 3 hours. After the completion of the reaction, the reaction mixture was washed by the addition of 200 ml of toluene and 50 ml of 0.5 N hydrochloric acid. The aqueous layer was removed, and the organic layer was then washed three times with 30 ml of distilled water. The solvent was distilled off to obtain 9,9-bis(methyl propionate)fluorene. Further, this compound was dissolved in 300 ml of isopropyl alcohol of 70°C and then recrystallized by cooling to 10°C to obtain 9,9-bis(methyl propionate)fluorene (FDP-m).

Table 1 shows results of evaluating light absorption characteristics as to various fluorene compounds obtained as described above.

**[Table 1]**

| | Substance | Maximum absorption wavelength λmax(nm) | Molar absorption coefficient [L/(mol·cm)] | | | Half width (nm) | 5% weight loss temperature [°C] |
|---|---|---|---|---|---|---|---|
| | | | λ=380nm | λ=400nm | λ=λmax | | |
| Example 1 | DNFDP-m | 340 | 752 | 2.76 | 63849 | 49 | 353 |
| Example 2 | DNFPO | 341 | 917 | 492 | 60801 | 49 | 387 |
| Example 3 | DNFPA | 340 | 701 | 322 | 66827 | 49 | 399 |
| Example 4 | DPFDP-m | 327 | 119 | 026 | 49152 | 52 | 287 |
| Example 5 | BNEF | 244 | <0.5 | <0.5 | 121564 | 20 | 401 |
| Example 6 | BPEF | 238 | <0.5 | <0.5 | 22997 | 20 | 333 |
| Example 7 | FDP-m | 267 | <0.5 | <0.5 | 14418 | 28 | 284 |
| Comparative Example 1 | Tinuvin 928 | 341 | 3881 | 160 | 17324 | 87 | 278 |
| Comparative Example 2 | LA-F70 | 355 | 27453 | 2010 | 67847 | 72 | 407 |

| | | | | | | | |
|---|---|---|---|---|---|---|---|
| Tinuvin 928: manufactured by BASF Japan Ltd., ultraviolet absorber LA-F70: manufactured by ADEKA Corp., ultraviolet absorber | | | | | | | |

All the fluorene compounds of Examples 1 to 7 were ultraviolet absorbers that had a small half width, were able to selectively absorb only ultraviolet ray in a specific range, and were able to achieve both an excellent UV absorbing property and a low staining property. Particularly, the fluorene compounds of Examples 1 to 3 had a large value of the molar absorption coefficient at 380 nm based on the molar absorption coefficient at 400 nm and can therefore achieve both an excellent UV absorbing property and a low staining property in a long-wavelength region. All the fluorene compounds had a higher 5% mass loss temperature than the existing benzotriazole-based ultraviolet absorber as shown in Comparative Example 1, indicating that their heat resistance were excellent.

### 3. Resin

### 3.1. Example 8

To 0.90 mol of BPEF, 2.10 mol of ethylene glycol (EG), 0.90 mol of FDP-m, and 0.10 mol of DNFDP-m, 2 × 10⁻⁴ mol of manganese acetate tetrahydrate and 8 × 10⁻⁴ mol of calcium acetate monohydrate were added as a transesterification catalyst, and the mixture was gradually melted by heating with stirring. After temperature elevation to 230°C, 14 × 10⁻⁴ mol of trimethyl phosphate and 20 × 10⁻⁴ mol of germanium oxide were added thereto, and EG was removed by gradual temperature elevation and pressure reduction until reaching 270°C and 0.13 kPa or lower. After reaching a predetermined stirring torque, the contents were isolated from the reactor to prepare ultraviolet absorbing polyester resin pellets.

As a result of analyzing the obtained pellets by 1H-NMR, 90% by mol and 10% by mol of diol components introduced in the ultraviolet absorbing polyester resin were derived from BPEFm and EG, respectively, and 90% by mol and 10% by mol of dicarboxylic acid components introduced therein were derived from FDP-m and DNFDP-m, respectively.

### 3.2. Examples 9 to 13

Each ultraviolet absorbing polyester resin was obtained in the same manner as in Example 8 except that the monomer composition of the diol component and the dicarboxylic acid component used was changed as shown in Table 2 below.

Table 2 shows the ratio of each constituent in the ultraviolet absorbing polyester resins described in Examples 8 to 13, etc.

**[Table 2]**

| | Ratio of each constituent of polymer | | | | (A₃₈₀/A₄₀₀) | Tg [°C] | Weight-average molecular weight (Mw) | 5% mass loss temperature [°C] |
|---|---|---|---|---|---|---|---|---|
| | Diol component | | Dicarboxylic acid component | | | | | |
| | BPEF [mol%] | EG [mol%] | FDP-m [mol%] | DNFDP-m [mol%] | | | | |
| Example 8 | 90 | 10 | 90 | 10 | 72 | 132 | 54900 | 409 |
| Example 9 | 85 | 15 | 80 | 20 | 96 | 135 | 70300 | 416 |
| Example 10 | 70 | 30 | 70 | 30 | 94 | 137 | 83700 | 410 |
| Example 11 | 50 | 50 | 50 | 50 | 109 | 136 | 92800 | 414 |
| Example 12 | 20 | 80 | 25 | 75 | 87 | 134 | 100600 | 422 |
| Example 13 | 70 | 30 | 0 | 100 | 70 | 156 | 98200 | 423 |

All the ultraviolet absorbing polyester resins of Examples 8 to 13 had a high ratio (A₃₈₀/A₄₀₀) of the molar absorption coefficient (A₃₈₀) to the molar absorption coefficient (A₄₀₀), indicating that these ultraviolet absorbers achieve both an excellent UV absorbing property in a long-wavelength region and a low staining property.

All the ultraviolet absorbing polyester resins had Tg of 130 to 160°C and a weight-average molecular weight that fell within the range of 50000 to 110000, were excellent in mechanical characteristics such as processability such as drawability, elongation at break, or flexibility, and further had a higher 5% mass loss temperature than that of the conventionally known benzotriazole-based and triazine-based ultraviolet absorbers, indicating that their heat resistance is also excellent.

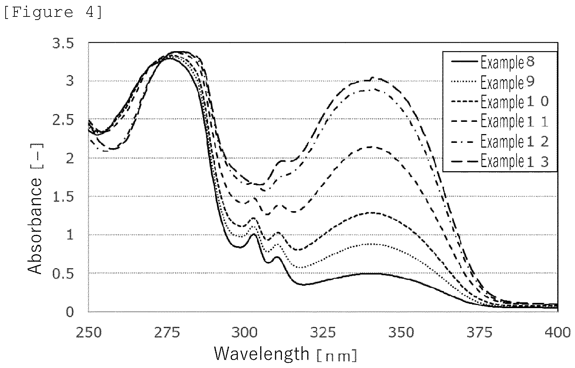

The ultraviolet absorbing polyester resins obtained in Examples 8 to 13 were dissolved in tetrahydrofuran to prepare their respective 50 mg/L solutions, followed by absorbance measurement. The evaluation results are shown in Figure 4. As shown in Figure 4, the ultraviolet absorbing polyester resins of Examples 8 to 13 exhibited high absorbance for ultraviolet ray in a wide wavelength band and exhibited low absorbance for visible light.

### 4. Resin composition and film

### 4.1. Examples 14 to 19 and Comparative Examples 3 to 6

### (Preparation of ultraviolet absorbing resin composition)

A dry blend of 95 parts by mass of dried pellets of the base polymer shown in Table 3 below (PC: polycarbonate, Lupilon S-3000, manufactured by Mitsubishi Engineering Plastics Corp., PMMA: polymethyl methacrylate, Parapet HR-S, manufactured by Kuraray Co., Ltd.) and 5 parts by mass of an ultraviolet absorber was supplied as a starting material to a twin-screw extrusion apparatus (manufactured by Technovel Corp., model "KZW 15/45", screw diameter D = 15 mm, L/D = 32) and kneaded at a screw temperature of 280°C and a rotational speed of 200 rpm to prepare an ultraviolet absorbing resin composition.

### (Preparation of ultraviolet absorbing film)

The ultraviolet absorbing resin composition obtained as described above was supplied to a twin-screw extrusion apparatus equipped with a T die, extruded into a film with the film thickness shown in Table 3 below, and taken up as a roll to prepare an ultraviolet absorbing film. Table 3 below shows results of evaluating each ultraviolet absorbing film. As shown in Table 3, the ultraviolet absorbing films of Examples 14 to 19 exhibited high ultraviolet absorption performance and a low staining property. These ultraviolet absorbing films can be suitably used, particularly, for polarizer protective film purposes.

**[Table 3]**

| | Base polymer | Ultraviolet absorber | Film thickness [µm] | Transmittance (%) at 350nm | L* | a* | b* |
|---|---|---|---|---|---|---|---|
| Example 14 | PC | DNFDP-m | 35 | 0.2 | 95.93 | -0.09 | 0.49 |
| Example 15 | PC | DNFPO | 50 | 0.1 | 95.97 | -0.09 | 0.50 |
| Example 16 | PC | DPFDP-m | 48 | 0.2 | 96.27 | -0.11 | 0.45 |
| Example 17 | PMMA | DNFDP-m | 53 | 0.1 | 97.58 | -0.11 | 0.22 |
| Example 18 | PMMA | DNFPO | 41 | 0.1 | 97.45 | -0.10 | 0.20 |
| Example 19 | PMMA | DPFDP-m | 63 | 0.1 | 97.69 | -0.11 | 0.17 |
| Comparative Example 3 | PC | Tinuvin 928 | 45 | 12.7 | 96.26 | -0.10 | 0.39 |
| Comparative Example 4 | PC | LA-F70 | 42 | 0.2 | 96.32 | -0.37 | 0.97 |
| Comparative Example 5 | PC | LA-F70 | 17 | 8.7 | 97.19 | -0.13 | 0.60 |
| Comparative Example 6 | PMMA | LA-F70 | 49 | 0.0 | 96.35 | -0.18 | 0.74 |

### 4.2. Examples 20 to 25

### (Preparation of ultraviolet absorbing film)

Each of the ultraviolet absorbing polyester resins obtained in Examples 8 to 13 mentioned above was supplied to a twin-screw extrusion apparatus equipped with a T die, extruded into a film with the film thickness of 50 µm, and taken up as a roll, followed by simultaneous biaxial drawing treatment under conditions of drawing temperature = Tg + 10°C using a tenter drawing apparatus to prepare an ultraviolet absorbing film with the film thickness shown in Table 4 below.

Table 4 below shows results of evaluating each ultraviolet absorbing film.

**[Table 4]**

| | Ratio of each constituent of polymer | | | | Film thickness | Transmittance (%) | |
|---|---|---|---|---|---|---|---|
| | Diol component | | Dicarboxylic acid component | | | | |
| | BPEF [mol%] | EG [mol%] | FDP-m [mol%] | DNFDP-m [mol%] | | 350nm | 380nm |
| Example 20 | 90 | 10 | 90 | 10 | 13 | 0.0 | 9.8 |
| Example 21 | 85 | 15 | 80 | 20 | 12 | 0.0 | 1.3 |
| Example 22 | 70 | 30 | 70 | 30 | 14 | 0.0 | 0.3 |
| Example 23 | 50 | 50 | 50 | 50 | 12 | 0.0 | 0.1 |
| Example 24 | 20 | 80 | 25 | 75 | 10 | 0.0 | 0.1 |
| Example 25 | 70 | 30 | 0 | 100 | 10 | 0.0 | 0.1 |

The ultraviolet absorbing films of Examples 17 to 22, even in the form of a 14 µm or smaller thin film, exhibited high ultraviolet absorption performance.

### Industrial Applicability

The ultraviolet absorber of the present invention has industrial applicability as a starting material for an ultraviolet absorbing coating liquid or composition, or a film obtained using the coating liquid or the composition.

### Reference Signs List

10 ... polarizer protective film, 20 and 30 ... polarizing plate, 22, 24, 32, 33, and 35 ... adhesive layer, 23 and 34 ... polarizer, 21 and 31 ... phase difference film, 40 ... organic EL display apparatus, 41 ... organic EL display panel, 42 ... touch sensor, 43 ... front panel, 50 ... liquid crystal display apparatus, 51 ... light source, 52 ... liquid crystal panel, 53 ... front panel

## Claims

1. An ultraviolet absorber being
a fluorene compound represented by the general formula (1) or a resin comprising the fluorene compound as a constituent unit: 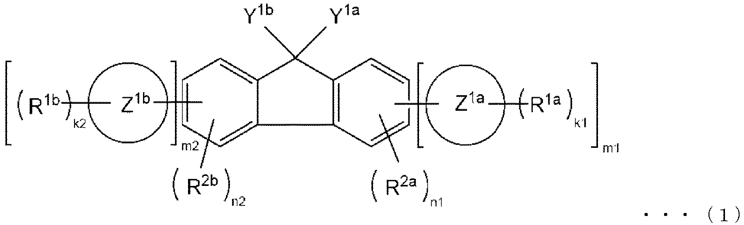 wherein
Z^{1a} and Z^{1b} each independently represent an arene ring,
Y^{1a} and Y^{1b} each independently represent a carboxy group, a carboxy ester group, an acid halide group, an acid anhydride group, a hydroxy group, or a group having a polymerizable double bond,
R^{1a} and R^{1b} each independently represent a halogen atom, an acyl group, a nitro group, a cyano group, a mono- or di-substituted amino group, -R^{A}, -OR^{A}, or -SR^{A} (on the proviso that R^{A} represents a hydrocarbon group),
k1 and k2 each independently represent an integer of 0 or larger,
m1 and m2 each independently represent an integer of 0 to 4,
R^{2a} and R^{2b} each independently represent a halogen atom, a cyano group, or a hydrocarbon group other than an aryl group,
n1 and n2 each independently represent an integer of 0 to 4, and
m1 + n1 and m2 + n2 are each independently an integer of 4 or smaller.

2. The ultraviolet absorber according to claim 1,
wherein
in the general formula (1), at least one of m1 and m2 is 1 or larger.

3. The ultraviolet absorber according to claim 1,
wherein
in the general formula (1), each of Z^{1a} and Z^{1b} is a condensed polycyclic arene ring, and both m1 and m2 are integers of 1 or larger.

4. The ultraviolet absorber according to claim 1, wherein
in the general formula (1), Y^{1a} and Y^{1b} are each independently a group represented by the formula (Y1) given below,
the fluorene compound is dicarboxylic acid or a derivative thereof, and
the resin is a polyester resin or a polyamide resin comprising the fluorene compound which is dicarboxylic acid as a constituent unit: 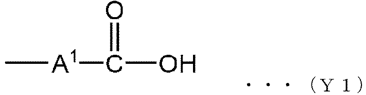 wherein A¹ represents a linear or branched C₁₋₆ alkylene group.

5. The ultraviolet absorber according to claim 1,
wherein
in the general formula (1), Y^{1a} and Y^{1b} are each independently a group represented by the formula (Y2) given below,
the fluorene compound is diol, and
the resin is a polyester resin or a polycarbonate resin comprising the fluorene compound which is diol as a constituent unit: 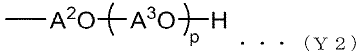 wherein
A² and A³ each independently represent a linear or branched C₁₋₆ alkylene group, and
P represents an integer of 0 or larger.

6. The ultraviolet absorber according to claim 1, wherein
in the general formula (1), Y^{1a} and Y^{1b} are each independently a group represented by the formula (Y3) given below,
the fluorene compound is diol, and
the resin is a polyester resin or a polycarbonate resin comprising the fluorene compound which is diol as a constituent unit: 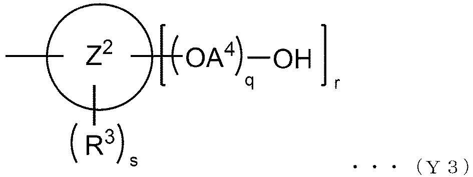 wherein
Z² represents an arene ring,
each A⁴ independently represents a linear or branched C₁₋₄ alkylene group,
each R³ independently represents a substituent,
q represents an integer of 0 or larger,
r represents an integer of 1 or larger, and
s represents an integer of 0 or larger.

7. The ultraviolet absorber according to claim 6, wherein
in the general formula (Y3), Z² is a condensed polycyclic arene ring.

8. The ultraviolet absorber according to claim 1, wherein
in the general formula (1), Y^{1a} and Y^{1b} are each independently a group represented by the formula (Y4) given below,
the fluorene compound is di(meth)acrylate, and
the resin is a (meth)acrylic resin comprising the fluorene compound which is di(meth)acrylate as a constituent unit: 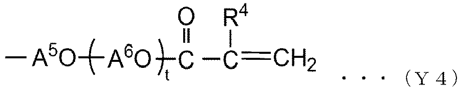 wherein
A⁵ and A⁶ each independently represent a linear or branched C₁₋₆ alkylene group,
R⁴ represents a hydrogen atom or a methyl group, and
t represents an integer of 0 or larger.

9. The ultraviolet absorber according to claim 1, wherein
a half width which is a wavelength band that indicates 1/2 or more of absorbance at a maximum absorption wavelength is 55 nm or less.

10. The ultraviolet absorber according to claim 1, wherein
the ultraviolet absorber has a maximum absorption wavelength within a wavelength range of 320 to 400 nm.

11. The ultraviolet absorber according to claim 1, wherein
a ratio (A₃₈₀/A₄₀₀) of a molar absorption coefficient at 380 nm (A₃₈₀) to a molar absorption coefficient at 400 nm (A₄₀₀) is 50 or more.

12. The ultraviolet absorber according to claim 1, wherein
a content of the constituent unit derived from the fluorene compound is 1 to 100% by mol based on all constituent units of the resin.

13. An ultraviolet absorbing composition comprising
the ultraviolet absorber according to claim 1, and
at least one or more compounds selected from a triazine ring-containing compound, a benzotriazole ring-containing compound, and a benzophenone ring-containing compound as a second ultraviolet absorber.

14. An ultraviolet absorbing resin composition comprising
the ultraviolet absorber according to claim 1, and
an arbitrary resin, wherein
a content of the ultraviolet absorber is 0.1 to 10% by mass based on the total amount.

15. An ultraviolet absorbing coating liquid, comprising
the ultraviolet absorber according to any one of claims 1 to 12, the ultraviolet absorbing composition according to claim 13, or the ultraviolet absorbing resin composition according to claim 14.

16. An ultraviolet absorbing coated resin sheet comprising
a resin sheet and an ultraviolet shielding layer, wherein
the ultraviolet shielding layer comprises the ultraviolet absorber according to any one of claims 1 to 12, the ultraviolet absorbing composition according to claim 13, or the ultraviolet absorbing resin composition according to claim 14.

17. An ultraviolet absorbing coated glass comprising
glass and an ultraviolet shielding layer, wherein
the ultraviolet shielding layer comprises the ultraviolet absorber according to any one of claims 1 to 12, the ultraviolet absorbing composition according to claim 13, or the ultraviolet absorbing resin composition according to claim 14.

18. An ultraviolet absorbing molded article comprising
the ultraviolet absorber according to any one of claims 1 to 12, the ultraviolet absorbing composition according to claim 13, or the ultraviolet absorbing resin composition according to claim 14.

19. An ultraviolet absorbing film comprising
the ultraviolet absorber according to any one of claims 1 to 12, the ultraviolet absorbing composition according to claim 13, or the ultraviolet absorbing resin composition according to claim 14.

20. A polarizer protective film comprising
the ultraviolet absorbing film according to claim 19.

21. The polarizer protective film according to claim 20, wherein
a spectral transmittance at 350 nm is 5% or less, and
a b* value in L*a*b* color space (CIELAB) is 0.5 or less.

22. The polarizer protective film according to claim 20, wherein
a spectral transmittance at 380 nm is 8% or less.

23. A polarizing plate comprising
the polarizer protective film according to claim 20.

24. An image display apparatus comprising
the polarizing plate according to claim 23.

25. Transport equipment comprising
the ultraviolet absorber according to any one of claims 1 to 12, the ultraviolet absorbing composition according to claim 13, or the ultraviolet absorbing resin composition according to claim 14.

26. A building member comprising
a member comprising the ultraviolet absorber according to any one of claims 1 to 12, the ultraviolet absorbing composition according to claim 13, or the ultraviolet absorbing resin composition according to claim 14.
